Europäisches Patentamt

European Patent Office

Office européen des brevets

(11) Publication number: **0 416 575 A2**

# EUROPEAN PATENT APPLICATION

(21) Application number: 90117068.8

(22) Date of filing: 05.09.90

(51) Int. Cl.5: **A61K 9/16, A61K 9/50,** A61K 9/20

(30) Priority: 05.09.89 US 403138
29.06.90 US 546947

(43) Date of publication of application:
13.03.91 Bulletin 91/11

(84) Designated Contracting States:
BE CH DE DK FR GB IT LI

(71) Applicant: MEDAPHORE INC.
540 Hunter Court
Wilmette, IL 60091(US)

(72) Inventor: Ecanow, Bernard
540 Hunter Court
Wilmette, Illinois 60091(US)

(74) Representative: Bauer, Robert, Dipl.-Ing. et al
Boeters & Bauer Patentanwälte
Bereiteranger 15
W-8000 München 90(DE)

(54) Drug delivery compositions based on biological materials and methods for preparing such compositions.

(57) Compositions and methods useful to introduce and transport one or more medically useful materials in the body of mammals by oral, tissue-absorptive, inhalation or parenteral administration include a medically useful material contained in particles of a coacervate-based matrix containing an egg portion selected from the group consisting of a portion of an egg white, a portion of an egg yolk, and mixtures thereof and having a structured surface on the particles.

EP 0 416 575 A2

# DRUG DELIVERY COMPOSITIONS BASED ON BIOLOGICAL MATERIALS AND METHODS FOR PREPARING SUCH COMPOSITIONS

The present invention relates to compositions useful to deliver drugs and other medically useful compositions into or, as required, on the bodies of humans and animals and the methods thereof. The components of biological cells, in whole or in part as exemplified by unfertilized, natural eggs of domestic fowl, preferably chickens, comprise principal and unique ingredients to prepare delivery compositions of this invention.

Finished drug products resulting from the use of the delivery systems of this invention can be prepared in any acceptable pharmaceutical dosage form, variation or combination thereof. Finished products of this method may be administered by any of the medically acceptable routes, variations or combinations thereof. Unlike most available drug delivery compositions, the delivery compositions of the present invention can effectively deliver parent compounds. In this method, parent compounds are the preferred starting drug components. Also, drugs now being formulated to an excessive pH can be prepared at or about the pH of the body through the use of the delivery compositions of this invention.

Effective delivery of medically useful compounds require formulations that overcome problems of solubility, stability and the like. Thus most drugs in common use are known to be lipid-soluble or preferentially so and poorly soluble in aqueous media. This property results in significant difficulty in the delivery of such drugs since the dosage formulation of such drugs generally requires the use of an essentially aqueous vehicle.

In order to overcome the poor aqueous solubility of such drugs, most lipid-soluble drugs are now formulated using the salt or other derivatives of the parent compound. To maintain solubility, many formulations require an excessive pH; this in turn often results in erratic delivery of the drug. Moreover, many current drug formulations are irritating to tissues and blood vessels when injected into the circulation. In addition, many lipid-soluble drugs are formulated in vehicles and co-solvents which in themselves may have irritant and toxic properties. Some formulations become diluted or neutralized when injected into the body causing formation of a precipitate of the drug and uneven absorption.

In the present invention, the delivery problems of lipid soluble drugs are overcome through the use of unique aqueous coacervate-based compositions based on the use of the yolk and/or the white of eggs and other compositions, as set forth in more detail hereinafter. A similarly based technology is disclosed herein for the preparation of encapsulated particles of hemoglobin useful for oxygen transport. The technology overcomes the toxicity problems associated with the use of unencapsulated hemoglobin for oxygen transport.

Unexpectedly, it has been found that the white and/or yolk of eggs, which contain more than thirty component ingredients constitute optimal starting materials for the manufacture of the compositions of this invention. Not all of these components are necessary to achieve the unexpected administration of physiologically-active materials in accordance with the present invention. This discovery represents a significant improvement over the inventor's previously disclosed use of albumin and lecithin. In accordance with the principals of the present invention, the use of eggs, particularly avian eggs as a starting material have several advantages; eggs are naturally hydrated, are relatively unvarying in their composition, are generally free of contaminants, or easily can be rendered so, and require no processing prior to use in this method or composition. Applications of this invention may make use of the egg white, and/or the egg yolk or combinations thereof in any proportions of egg white to yolk preferred by the formulator.

It is understood that in this specification any reference to the use of eggs or their components, derivatives, and the like, is used to mean that the principal source thereof is unfertilized avian and the like, particularly chicken eggs, in their natural state. In addition, while the preferred source is chicken eggs, this invention includes the manufacture of formulations in which the unfertilized natural ova in whole or in part of any pharmaceutically acceptable species, e.g., avian, picean and the like may be used. The use of fertilized ova of acceptable species is also useful in accordance with the present invention.

The yolk of the natural unfertilized chicken egg comprises an excellent starting composition from which to prepare emulsions, microemulsions, suspensions and the like, of lipid-soluble drugs, or drugs that are preferentially soluble in lipids, and of liposome-encapsulated drugs. Egg yolk is also a useful starting material to prepare the red blood cell substitute and oxygen carrying fluids based thereon; said fluids being useful for resuscitation in conditions of significant blood loss, carbon monoxide poisoning, and the like; for perfusion of organs prior to transplant; for angioplasty and other cardiac treatment procedures and for use in cell and tissue culture media.

Egg white unexpectedly has been found to be an effective solubilizing agent for water-insoluble drugs and a useful composition to prepare red blood cell substitutes. The method of this invention is also useful to improve the stability and delivery characteristics of emulsions and suspensions of drugs prepared by other methods known to the art.

In addition to the drug delivery system of this invention, the methods thereof are useful to produce the following oxygen carrying compositions: a non-toxic microencapsulated red blood cell substitute; a resuscitation fluid; a fluid useful to perfuse organs and tissues; and a medium useful for cell and tissue culture. The synthetic red cell of this invention is modeled after its human counterpart. It is unique in that it is comprised of a coacervate-based matrix which incorporates all of the constituent components of the natural red blood cell cytoplasm including hemoglobin and a coacervate-based encapsulating film.

The encapsulating film of the disclosed synthetic red cell prevents untimely release or exposure of free hemoglobin during the dwell time of the composition in the circulation. This feature is of special clinical importance since exposure or release of free hemoglobin is known to be associated with endotoxic and free radical toxicities. The literature also reports iron toxicities resulting from the presence of free hemoglobin in the blood.

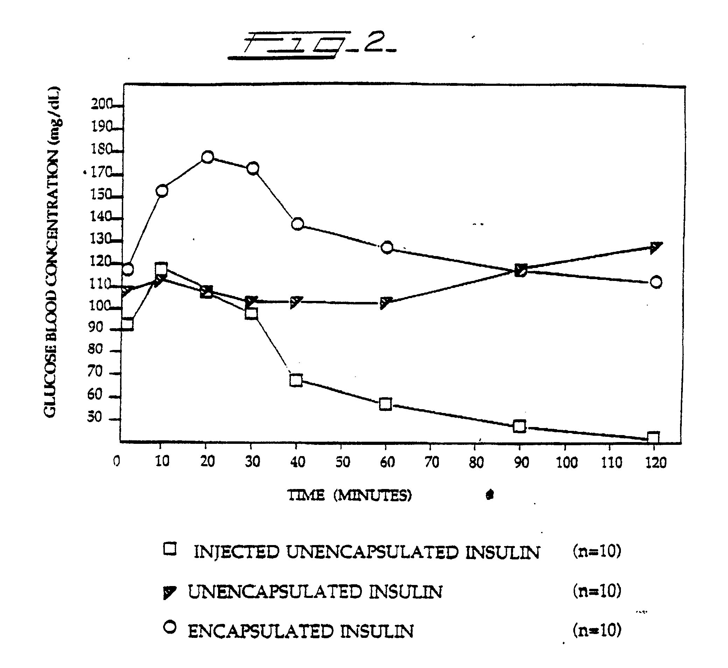

The compositions and methods of the present invention are particularly useful for the oral administration of any and all forms of insulin, from every source, as indicated in the drawings. One particularly new and unexpected property of the compositions and methods of the present invention is that the compositions prepared in accordance with the present invention that contain insulin as the physiologically-active component will, in a short time, level the glucose level in the blood stream of a mammal that is experiencing a rapid rise in glucose, as shown in Fig. 2.


## PRIOR ART


The inventor's prior art includes the following: U.S. Patent No. 4,343,393 disclosed a method of preparing a synthetic blood substitute based upon a two-phase heterogeneous physico-chemical composition that provides oxygen transport and other physiological functions inherent to whole natural blood; U.S. Patent No. 4,439,424 comprises a synthetic blood composition based on albumin, water, sodium chloride, phospholipid and stroma-free hemoglobin; U.S. Patent No. 4,558,032 discloses a synthetic blood comprised of gelatin, acacia and stroma-free hemoglobin; U.S. Patent No. 4,539,204 discloses a synthetic blood comprised of stroma-free hemoglobin, two gelatins, modified fluid gelatins and lecithin; U.S. Patent No. 4,547,490 disclosed a coacervate-based synthetic blood composition derived from an aqueous solution containing albumin, sodium chloride and lecithin in a nonpolar or semipolar solvent; and U.S. Patent No. 4,849,405 discloses a method of administering insulin orally from a coacervate composition.

Additional pending applications by the inventor include Serial No. 896,844 filed August 14, 1986; Serial No. 001,814 filed January 8, 1987, Serial No. 031,237 filed March 26, 1987, Serial No. 054,193 filed May 26, 1987; Serial No. 054,194 filed May 26, 1987; and Serial No. 267,749 filed November 2, 1988.

U.S. Patent No. 4,397,870 discloses an emulsified lecithin-coated perfluorocarbon composition. This composition is unrelated both in terms of its components and its physical pharmacy form to the coacervate compositions of the present invention. Moreover, perfluorocarbons have been reported to have toxic potential, unlike the compositions of the present invention. U.S. Patent No. 4,652,257 discloses a drug delivery composition based on the use of magnetic fields and the use of polymerized lipids. The products of the present invention are significantly different in composition and method. U.S. Patent No. 4,133,874 discloses the use of liposomes. Liposomes bear very little relationship to the coavervate compositions of the present invention or physical-chemical characteristics of coacervates.

Modified hemoglobin-based blood substitutes developed by DeVenuto, Moss, Gould and others, Fronticelli and Greenberg are described in the literature (Ref. Advances in Blood Substitute Research, Alan Liss, New York, 1983). The same reference describes perfluorocarbon-based blood substitutes. These prior art blood substitutes have very little compositional similarity or similarity of method to the herein disclosed red blood cell substitutes and related products.

The present invention is directed to methods and compositions, preferably coacervate-based microencapsulated compositions that provide for the delivery of medically useful products such as drugs, biologicals and the like into and, as desired, on the body of humans and animals. The disclosed compositions not only comprise effective delivery systems for drugs and other medically useful compositions such as nutrients, micro-nutrients, vitamins and the like but when desired, these systems can be prepared in a manner that will also overcome offensive odors and tastes of drugs and nutrients, vitamins, and the like making the product more palatable. In such application, the product is encapsulated by the disclosed method, thereby rendering it virtually free of taste or odor. As an option, conventional flavoring agents can be added to the composition following this processing step.

In another application of this method a synthetic "red cell" is produced. Oxygen carrying fluids based thereon, including fluids useful for resuscitation, organ perfusion, angioplasty and other cardiac treatments are also provided for in this invention. An oxygen carrying cell culture medium also can be produced through the methods of this invention.

The finished products of this invention may be in any of the following forms: emulsions, suspensions, microsuspensions, microemulsions, gels, powders and other pharmaceutical dosage forms. To achieve the full advantage of the present invention, particle size is an important feature. In the practice of this invention particle size is adapted to the function of the product. Accordingly, formulations of this method may be comprised of particles ranging from about 0.125 microns to about 100 microns or more.

Finished products based on the use of this invention are stable, effective and pharmaceutically acceptable.

## THE CONSTITUENTS

Methods and compositions of the present invention are based on the use of eggs in whole or in part from domestic fowl, more particularly, said methods are based on the use of egg yolk and/or egg white of chicken eggs, in whole or in part, or derivatives thereof, in liquid or powder form, or mixtures thereof without limit. Portions of the egg white and/or yolk can be removed, such as the lipids, minerals, non-essential proteins, vitamins and/or carbohydrates, or any portion thereof, in any combination while achieving the excellent drug delivery results in accordance with the principles of the present invention. These compositions are used as starting materials to produce aqueous compositions which are subsequently processed in the manner described below to produce microencapsulated particles containing one or more physiologically-active compounds. For purposes of this invention, the preferred egg white and egg yolk constituents and concentrations thereof are given in the following TABLE I. (Ref. McGraw-Hill, Encyclopedia of Food, Agriculture and Nutrition, 1978, p. 225).

TABLE I

| Composition<br><br>Nutrients per<br>gram of product | Whole Eggs | | Egg White | | Egg Yolk | |
|---|---|---|---|---|---|---|
| | Fresh<br>or<br>frozen | Solids | Fresh<br>or<br>frozen | Solids | Fresh<br>or<br>frozen | Solids |
| Calories | 163 | 606 | 51 | 412 | 312 | 656 |
| Water, g | 74..5 | 5.0 | 88.6 | 8.0 | 55.0 | 5.0 |
| Protein, g | 12.0 | 44.6 | 10.0 | 80.7 | 14.0 | 30.0 |
| Amino acids, g | | | | | | |
| Arginine | 0.80 | 2.99 | 0.63 | 5.08 | 1.01 | 2.13 |
| Aspartic acid | 0.70 | 2.59 | 0.60 | 4.84 | 0.77 | 1.62 |
| Cystine | 0.26 | 0.98 | 0.25 | 2.02 | 0.24 | 0.51 |
| Glutamic acid | 1.48 | 5.50 | 1.24 | 10.01 | 1.69 | 3.57 |
| Glycine | 0.44 | 1.65 | 0.40 | 3.23 | 0.49 | 1.03 |
| Histidine | 0.32 | 1.20 | 0.27 | 2.18 | 0.41 | 0.87 |
| Isoleucine | 0.84 | 3.12 | 0.72 | 5.81 | 0.97 | 2.05 |
| Leucine | 1.02 | 3.79 | 0.85 | 6.86 | 1.19 | 2.51 |
| Lysine | 0.82 | 3.03 | 0.66 | 5.33 | 1.01 | 2.13 |
| Methionine | 0.40 | 1.47 | 0.41 | 3.31 | 0.34 | 0.72 |
| Phenylalanine | 0.65 | 2.41 | 0.61 | 4.92 | 0.64 | 1.35 |
| Serine | 0.92 | 3.43 | 0.69 | 5.57 | 1.25 | 2.64 |
| Threonine | 0.66 | 2.46 | 0.52 | 4.20 | 0.85 | 1.79 |
| Tryptophan | 0.23 | 0.85 | 0.20 | 1.61 | 0.25 | 0.53 |
| Tyrosine | 0.55 | 2.05 | 0.46 | 3.71 | 0.64 | 1.35 |
| Valine | 0.98 | 3.66 | 0.88 | 7.10 | 1.02 | 2.15 |
| Lipids, g | 11.0 | 40.8 | --- | --- | 28.9 | 60.7 |
| Cholesterol | | | | | | |
| Fatty acids, g | 3.6 | 13.1 | --- | --- | 9.7 | 18.1 |
| Monounsaturated | 6.2 | 22.3 | --- | --- | 16.6 | 30.6 |
| Polyunsaturated | 1.4 | 5.1 | --- | --- | 3.8 | 6.9 |
| Saturated | 0.8 | 2.9 | --- | --- | 1.8 | 3.9 |
| Phospholipids | 3.3 | 12.4 | --- | --- | 9.0 | 20.0 |
| Minerals (ash), g | 1.0 | 3.7 | 0.7 | 5.7 | 1.6 | 3.4 |
| Calcium, mg | 54.0 | 201.0 | 6.0 | 48.4 | 147.0 | 309.0 |
| Chlorine, mg | 100.0 | 372.0 | 131.0 | 1057.0 | 67.0 | 141.0 |
| Copper, mg | 0.17 | 0.63 | 0.04 | 0.32 | 0.25 | 0.52 |
| Fluorine, mg | 0.06 | 0.22 | 0.02 | 0.16 | 0.12 | 0.25 |
| Iodine, mg | 12.0 | 45.0 | 6.8 | 54.9 | 16.0 | 34.0 |
| Iron, mg | 2.1 | 7.8 | 0.3 | 0.24 | 5.6 | 11.8 |
| Magnesium, mg | 9.0 | 33.5 | 11.0 | 89.0 | 13.0 | 27.0 |
| Manganese, mg | 0.04 | 0.15 | --- | --- | 0.11 | 0.23 |
| Phosphorus, mg | 210.0 | 781.0 | 17.0 | 137.0 | 586.0 | 1231.0 |
| Potassium, mg | 149.0 | 554.0 | 149.0 | 1202.0 | 110.0 | 231.0 |
| Sodium, mg | 111.0 | 413.0 | 175.0 | 1412.0 | 78.0 | 164.0 |
| Sulfur, mg | 233.0 | 867.0 | 211.0 | 1702.0 | 214.0 | 449.0 |
| Zinc, mg | 1.3 | 4.8 | 0.01 | 0.8 | 3.8 | 8.0 |
| Vitamins | | | | | | |
| A, IU | 1140.0 | 4240.0 | --- | --- | 3210.0 | 6741.0 |
| $B_{12}$, ug | 0.28 | 1.04 | 0.01 | 0.08 | 0.83 | 1.74 |
| Biotin, ug | 22.5 | 83.7 | 7.0 | 56.5 | 52.0 | 109.0 |

| Composition Nutrients per gram of product | Whole Eggs Fresh or frozen | Solids | Egg White Fresh or frozen | Solids | Egg Yolk Fresh or frozen | Solids |
|---|---|---|---|---|---|---|
| Choline, g | 0.53 | 1.97 | --- | --- | 1.49 | 3.13 |
| D, IU | 50.0 | 186.0 | --- | --- | 150.0 | 315.0 |
| E, mg | 2.0 | 7.4 | --- | --- | 6.0 | 12.6 |
| Folic acid, mg | 9.4 | 35.0 | 1.6 | 12.9 | 23.2 | 48.7 |
| Inositol, mg | 33.0 | 122.8 | --- | --- | --- | --- |
| Niacin, mg | 0.1 | 0.37 | --- | --- | --- | --- |
| Pantothenic acid, mg | 2.7 | 10.0 | 0.13 | 1.05 | 6.0 | 12.6 |
| Pyridoxine, mg | 0.25 | 0.93 | 0.22 | 1.78 | 0.31 | 0.65 |
| Riboflavin, mg | 0.29 | 1.08 | 0.26 | 2.1 | 0.35 | 0.74 |
| Thiamine, mg | 0.1 | 0.37 | --- | --- | 0.27 | 0.57 |
| Carbohydrates, g | 0.7 | --- | 0.8 | --- | 0.7 | --- |

As an option, individual constituents of the egg and their concentrations may be modified prior to use, that is, as preferred by the formulator, specific constituents may be extracted and eliminated or quantities of individual constituents may be increased or decreased. Frozen and thawed eggs or its components, reconstituted dried eggs or components are among the optional substitutes for the preferred natural egg while and egg yolk. Eggs that have been subjected to "pasteurization" processes are acceptable for this method. Also, synthetic equivalents of egg yolk and egg white may be used in this method.

It is preferred that egg yolk and egg white be used directly as they are obtained from the whole egg to prepare compositions of this method. As an option, however, egg yolk and egg white may be filtered using any appropriate filtering method and filter sizes, e.g., from 1 micron or less to filter out pathogens and 1 micron or larger.

The egg yolk and egg white components and derivatives thereof may be used in either liquid or lyophilized form or acceptable mixtures thereof. As an option wherein egg yolk is used either singly or in combination with egg white to prepare drug delivery systems of this method, a phospholipid may be added to the egg yolk, substituted for it, or used in combination with it. Suitable phospholipids include the following without limitation: natural and synthetic lecithins, coacervated lecithin, cephalins, and sphingomyelins, as well as phosphatidic acid, phosphatidyl serine, phosphatidyl inositol, phosphatidyl choline, and phosphatidyl ethanolamine, egg phosphatides, and combinations thereof. If desired, lyophilized or coacervated forms or combinations of these compositions may be used.

In this method, egg yolk and, as desired, phospholipids or combinations thereof are used as emulsifying and stabilizing agents and as film forming compositions.

As an option in this method wherein egg white is one of the components of given formulations, a suitable proteinaceous composition or combination of proteins may be used in combination with egg white, substituted for it, so long as used with the egg yolk or substantial portion thereof. Suitable proteins including compounds such as albumins; globulins; glutelins; gelatins; modified fluid gelatins; prolamines; albuminoids; histones; protamines; conjugated proteins, including phosphoproteins, nucleo-proteins, glycoproteins; chromoproteins; lipoproteins; metaloprotein and derived proteins, such as proteans; metaproteins, conjugated proteins, proteoses, peptones, or peptides; polypeptides; and/or the known collagens and combinations thereof. In the method of this invention, albumin or other proteins present in egg white may be a component of encapsulating films.

Additional compositions used in the methods of this invention include one or more of the following, either singly or in combinations. As an option, saccharides may be used as stabilizing agents, particularly of the hemoglobin component used to prepare the disclosed red cell substitutes.

As used in this specification, the saccharides include disaccharides, oligosaccharides, and polysaccharides. Included without limitation in the monosaccharides are dextrose, fructose, and glactose and the sugar alcohols such as mannitol, xylitol, inositol and sorbitol. Suitable saccharides that may be used, without limitation, include sucrose, lactose, and maltose. Among these optional compounds, sucrose is preferred. Suitable oligosaccharides, without limitation, include polymers of the monosaccharide sugars containing from about 3 to about 6 monomer units. Polysaccharides without limitation, include gelling polysacchardies, such as the alginate composition, particularly sodium alginate, and include dextrins having weight average molecular weights ranging from 19,500 or less to approximately 70,000 or higher;

hydroethyl starch and the cyclo-dextrins are included among other polysaccharide compounds useful in the method of this invention. This invention also includes formulations based on hydroethyl starch and the disclosed synthetic red cells. Such formulations are useful as circulation volume expanders with oxygen transport capability.

Dextrins with weight average molecular weights of about 19,500 to less than about 40,000 are preferred for formulations of this method. For red blood cell substitute formulations, dextrin having a weight average molecular weight of about 19,500 is preferred. As an option, a combination of dextrins with differing molecular weights may be used. In the method of this invention, dextrins are used as film forming agent.

Compositions, particularly surfactant agents, are used to prepare two phase coacervate compositions or coacervate-based matrix and encapsulating film compositions which are basic to the method of this invention. These compositions are used as solvents, and also to form particle matrices and particle encapsulating films. The surfactants which may be used singly or in any pharmaceutically acceptable combination in this invention are known in the literature as anionic agents, cationic agents, non-ionic agents and amphoteric agents. (Reg.: Remington's Pharmaceutical Sciences, 1976.) Remington's Pharmaceutical Sciences, pp. 295-297 is hereby incorporated by reference.

Preferred applications of this method use the coacervate (colloid rich) phase or the equilibrium water (colloid poor) phase or both phases of the disclosed coacervate systems. In this invention, the density of the coacervate phase may be greater or less than that of the equilibrium phase. As used in this specification, the term "colloid rich" is used to refer to the aqueous coacervate phase of the coacervate system; the "colloid poor" phase is used to refer to the aqueous equilibrium water phase of said system.

Pharmaceutical solvent and co-solvent compositions singly or in combination are used in the methods of the present invention pertaining to drug delivery systems. These include without limit, pharmaceutically acceptable polar, semi-polar and non-polar solvents such as essential fatty acids, such as linoleic acid, other fatty acids, water, ethyl alcohol, benzene, chloroform, acetone, and the like; also, oils derived from plant and vegetable sources including components thereof such as seeds, nuts and the like, i.e., peanuts, olives, and the like and seeds from cotton, safflower flax, soybean, corn, sesame and combinations thereof. Other solvent and co-solvent compositions suitable for use in this invention include mono, di, and triglycerides, or mixtures and blends thereof as preferred by the formulator.

Other lipids which are acceptable for the method of this invention include synthetic and semi-synthetic mono, di- and/or triglycerides or glycerides prepared by fractionization of natural synthetic or semi-synthetic triglycerides and triglycerides prepared by esterification.

Fatty compounds, i.e., natural or synthetic fatty acid esters of glycerol, as exemplified by tristerol glyceride category of acceptable optional solvents, comprise another category of solvents useful in the practice of the present invention. In this invention, triglycerides comprise the preferred solvent or co-solvent compositions.

Glycol ethers and their derivatives, singly or in optional combination also may be used as solvents and co-solvents in the practice of the present invention. The preferred glycol is polyethylene glycol with a weight average molecular weight of about 8,000. Polyethylene glycols with molecular weights ranging from about 190 to 9,000 may be used as optional or additional solvents.

A sterol, such as cholesterol, may be used as an option in this method in place of or in conjunction with the described vegetable oils. In the synthetic "red cell", formulations of this method, cholesterol may be used as an optional stabilizing agent. In another optional use of a sterol, particularly in the oral drug delivery formulations of this invention, bile salts and the like also may be used to facilitate drug absorption. Urea may be used in synthetic "red cell" formulations as an optional ingredient.

Derivatives of cellulose such as ethyl, methyl and propyl cellulose are among the compounds used in this method to produce finished drug compositions with sustained release characteristics.

Carnauba wax also can be used in this method as an optional component of the drug delivery formulations to give sustained release and other characteristics to finished drug delivery products.

As required, non-toxic preservatives without limit, such as propyl paraben, methyl paraben, or combinations of these and other pharmaceutically acceptable preservatives may be added during the formulation processes of this method. Preservatives may be added to finished drug products in amounts ranging from trace amounts up to about 1% by weight or more, as preferred by the formulator. Any non-toxic alcohol, but particularly ethyl alcohol, may be used as a protein-precipitating agent according to this method.

Nutrient compositions which may be delivered by means of the methods and compositions of the present invention may include water-soluble and water-insoluble vitamins, trace elements, carbohydrates, amino acids and related compounds, electrolytes and mixtures thereof according to the requirements of the formulator. Adjuvant compositions are also included in parenteral formulations to prevent oxidation, i.e., a tocopherol such as vitamin E; and compounds to adjust the isotonicity and pH to normal values. Carnitine comprises another optional additive.

Preferably, any amino acids used in the compositions of the present invention are branched chain amino acids because of their reported beneficial effect on plasma amino acid levels. Compositions related to amino acids, such as keto analogs, oligo and polypeptides of synthetic origin also may be used.

Water-soluble compounds referred to above include vitamin C, the B vitamins; and water-soluble trace elements including chromium, iron, copper, calcium, magnesium, zinc and selenium.

Carbohydrates that may be used in the methods and compositions of the present invention include monosacchardies, disacchardies, oligosaccharides and polysaccharides without limit and mixtures thereof as preferred by the formulator.

Water-insoluble vitamins include vitamin A and vitamin D and are prepared in this formulation through the use of the egg yolk or, preferably, the colloid rich phase of the coacervate compositions described previously.

An oxygen transport compound, such as stroma-free hemoglobin or as an option, any pharmaceutically-acceptable combination of oxygen carrying compounds, in any proportions preferred by the formulator can be included in the compositions of the present invention to manufacture the red cell substitute. In this method, the preferred oxygen transport compound is hemoglobin. The sources of hemoglobin may be human, bovine or hemoglobin derived from genetic engineering or other synthetic processes, or any combination thereof and include stroma-free hemoglobin and its derivatives and polymers, such as polymerized, pyridoxilated and pyridoxilated-polymerized hemoglobin; crosslinked stroma-free hemoglobin; also hemoglobin linked to, or contained in other carriers, such as liposomes, or active compounds of hemoglobin polymers; also sterilized stroma-free hemoglobin, its derivatives and polymers; and stroma-free hemoglobin prepared according to the method of this invention. Stroma-free hemoglobin or the heme of hemoglobin or combinations thereof are used in this method to prepare oral hematinics.

By reason of the inclusion of red cell cytoplasm components in the synthetic "red cell" of this invention, the monomeric hemoglobin acts as tetrameric hemoglobin. Electrolytes are used in the preparation of the disclosed resuscitation fluid. Electrolytes are added as desired in amounts sufficient to render the composition isotonic with whole human blood. As preferred by the formulator, electrolytes in pharmaceutically appropriate amounts may be added to the organ perfusion fluid, described in more detail hereinafter. In the manufacture of the resuscitation and perfusion fluids, the following components may be used single or in any combination without limitation: sodium, potassium, magnesium, calcium, lithium, ammonium, phosphorous, chlorides, iodides, fluorides, sulfates, carbonates, phosphates, lactates, acetates, gluconates and lactobionates.

Enzymes known to facilitate oxygen transport and release also may be included in the resuscitation fluid compositions of the present invention. The enzymes, when added, are present in the stroma-free hemoglobin solution prepared according to the method of the present invention. As preferred by the formulator, enzymes used singly or in combination may be added to the preparation during the manufacture of synthetic "red cells". These enzymes include, without limit, enzymes of the glycolic system such as adenosine diphosphate, adenoxine triphosphate, catalase, carbonic anhydrase, and the like. Anti-oxidants present in the human red cell such as glutathione as well as other natural and non-toxic synthetic antioxidants, without limit, such as ascorbic acid; alpha tocopherols, such as vitamin E; nicotinamide adenine; nicotinamide dinucleotide phosphate, and the like, can be used singly or in combination as preferred by the formulator to make the "red cell" of this invention.

It is preferred that about 95% or more of the iron in the hemoglobin be in the ferrous state when used to prepare the red blood cell substitute of this method. If, during manufacturing or storage steps, the ferrous iron fraction of the hemoglobin is less than the preferred quantity, the composition may be processed as follows to restore the ferrous state to about 95% or more of iron present. The hemoglobin, or the composition containing the hemoglobin, is placed in an electron donating chamber and subjected to an electrical current until measurement indicates that the hemoblogin contains about 5% or less or iron in the ferric state (methemoglobin). In this process, the electircal current can range from less than 1/2 volt ot 120 volts or more. An electrical current of about 9 volts is preferred.

This invention also contemplates the use of the above-described electrical processing method to maintain desirable ferrous iron fractions in liposome-encapsulated hemoglobin preparations. The herein-described electrical processing step of hemoglobin may also be applied as required to other compositions of this method that contain hemoglobin, such as the resuscitation fluid, the organ perfusion fluid and the cell culture media, described in more detail hereinafter.

It is anticipated in this method that the disclosed electrical processing steps may be applied as required to synthetic "red cells" which contain hemoglobin polymers or derivatives thereof, or combinations thereof. Such application may be made when it is necessary to reduce the $Fe^{+3}$ of the hemoglobin polymer to $Fe^{+2}$

to promote oxygen transport. The described electrical processing method has other applications important in this invention.

As desired, the synthetic "red cells" are subjected to the electrical process described above to restore enzymes contained therein to a more active state (e.g., -SH, not -SS-). The process will also act to remove free radicals and peroxides from the hemoglobin and eliminate unsaturated molecules from the encapsulating film of the disclosed "red cell" substitute.

In the method of this invention, the electrical process described above also may be used to preserve the viability of frozen natural red blood cells and frozen natural whole blood without the need for a polyhydric alcohol, such as glycerin. In this embodiment, the process used can be identical to that used for processing the synthetic "red cell".

## CHARACTERISTICS OF THE SYNTHETIC "RED CELL" (Synthocytes)

The red cell substitute prepared in accordance with the method of the present invention has oxygen uptake and release and oxygen-carbon dioxide exchange characteristics virtually identical to those of its natural counterpart and is the basic constituent of the oxygen carrying compositions of the present invention.

When an appropriate quantity of the red cell substitute of the present invention is dispersed in a suitable fluid, such as physiological saline solution, and the pH, isotonicity, and viscosity of the composition are adjusted to approximate normal standards, the product will transport and release oxygen, and restore and maintain normal oncotic pressure in mammals, particularly humans. The interfacial tensions of this product range from about 1 to about 5 dynes, thereby enabling it to traverse the microcirculation and narrowed or collapsed vessels of the vascular system. Circulatory dwell time of the product can range from about 6 to about 72 hours or more, as required. Dwell time can be modified, using the processes of the present invention to produce the desired particle size and encapsulating film structure. When the described synthetic "red cells" are dispersed in an appropriate liquid vehicle, such as Ringer's solution or saline solution, the equilibrium water phase of the coacervate compositions of this invention is included in the composition in an appropriate quantity, and the product is adjusted for pH and isotonicity, to provide a resuscitation fluid useful for restoring or maintaining normal oxygen transport and oncotic pressures to recipients.

In accordance with another important feature of the present invention, the resuscitation fluid also can be used as a perfusion fluid capable of carrying sufficient oxygen and other physiologically useful components so as to extend the viability of organs and tissues stored for transplant purposes.

As desired, the perfusion fluid may be used in conjunction with equipment designed to circulate fluid through organs in storage or may be used as the storage vehicle per se.

In one embodiment of the method of this invention, red blood cells of the present invention can be added to produce new, oxygen-carrying cell culture media suitable for culturing normal and pathological cells of animal and human sources.

In accordance with another important embodiment of the present invention, any suitable ferrous hemoglobin compound processed according to the methods of this invention, preferably the disclosed red cell substitute, may be dispersed in any medically suitable quantity, in any acceptable oral vehicle, particularly the colloid rich phase of the coacervate compositions of the present invention and dispensed as an oral hematinic. If desired, any acceptable ferrous compound processed by the method of the present invention can be used in place of, or in combination with, the ferrous iron of hemoglobin to produce an oral hematinic composition. These ferrous compounds include the gluconate, sulfate, lactate, and succinate salts, or any combination thereof.

A number of terms used throughout this specification are defined below. The methods of this invention involves the use of compositions derived through the process of coacervation. For purposes of this specification, coacervation is defined to mean the process by which a single phase aqueous solution is processed with one or more surfactants or other coacervating agents so that the aqueous solution separates into two or more liquid aqueous layers in equilibrium; said layers comprise a coacervate system or composition. The layers (or phases) can be separated, as appropriate; the layers are useful as carrier vehicles for an active component; as solvent systems and/or as film forming compositions. In some formulations described below one phase is used in preparing the compositions; in other compositions, both phases are used. Formation of layers from the solution occurs as one of the layers becomes colloid rich. This layer is referred to as the coacervate phase. The other layer in equilibrium with the colloid rich layer is colloid poor and is known as the equilibrium water phase. The coacervate phase comprises a non-polar composition composed predominantly of "structured" water and contains most of the white and/or yolk, surfactants and other components as are added to the starting bulk water. In this method, the equilibrium water phase comprises a polar composition composed predominantly of water and containing a small proportion of egg white and/or yolk and surfactant and other components as are added to the water solution. The coacervate phase may be amorphous in nature or it may be crystalline in organization (sometimes referred to as liquid crystals); the equilibrium water phase of this invention is used primarily in formulations containing polar and water-soluble active components.

The term "drug" is used in accordance with the definition by the Federal Food, Drug and Cosmetic Act, which defines the term as, "articles recognized in the official United States Pharmacopaeia, Homeopathic Pharmacopaeia, or official National Formulary or any supplement to any of them." As used here, "drug" also follows the definition of the Pennsylvania Pharmacy Act, which states, "the term 'drug' means (1) articles recognized in the official United States Pharmacopaeia, official Homeopathic Pharmacopaeia of the United States, or official National Formulary, or any supplement to any of them, intended for use in the diagnosis, cure, mitigation, treatment, or prevention of disease in man or other animals; and (2) all other articles intended for use in the diagnosis, cure mitigation, treatment, or prevention of disease in man or other animals; and (3) articles intended to affect the structure or any function of the body of man or other animals; and (4) articles intended for use as a component or any articles specified in clause (1), (2), (3); but does not include devices or their components, parts, or accessories." (Uniform State Pharmacy Act) (Reference: Remington's Pharmaceutical Sciences, 1976, p. 1843).

The term "drug" as used herein includes without limit lipid-soluble drugs, drugs which are preferentially soluble in lipids, water-soluble drugs and drugs requiring an organic or other solvent(s) for solubilization or stabilization before processing according to the methods of the prsent invention. The term "particles" as used herein refers to encapsulated or unencapsulated coacervate-based particles comprised of a matrix containing the coacervate phase, the equilibrium phase or mixtures thereof of a coacervate system in which the physiologically-active compound or other compounds, generally optional, e.g., a sterol, is solubilized or dispersed. The outer surface of the particles may have the same or greater degree of structure than the interior of the particle. In this method, compositions may be comprised of particles ranging in size from submicron, e.g., .001 micron to 100 microns or more in any single dimension.

The term "biological" is used to mean any compound or composition that may be usefully administered to treat or diagnose disease states and is derived directly or indirectly from a biological source of synthetic equivalent.

As used herein, the terms "oral", "transdermal", "parenteral", "opthalmic", and "inhalation" are used in accordance with conventional definitions. In this specification, use of the term "opthalmic" does not imply that the finished products introduced to the body by this method and route are restricted to delivery of drugs useful to treat conditions of the eye. Similarly, in this invention, the inhalation mode of delivery is not restricted to drugs and other compositions now commonly administered by inhalation. This invention fully anticipates inhalation formulations to deliver a wide array of drugs such as antibiotics, insulin, other polypeptides and other drugs now administered by other means particularly by injection.

In this specification, parenteral formulations are administered by injection and include without exception all of the known injection routes. In addition, this invention includes parenteral formulations which are known as large volume parenteral dosage forms, the volume of which may range from about 100 mls to about 3000 mls of fluid and are usually administered by means of a single infusion. This specification also includes parenteral compositions known as small volume parenteral dosage forms, the volume of which may range from about 0.1 ml to about 100 ml of fluid. The practice of this invention provides for the addition of small volume parenteral dosage forms to large volume dosage forms. Such addition provides diluted but continuous administration of the drug.

"Tissue absorptive" refers to the capability of the composition to be absorbed by application in the form of a gel, cream, solution, or other suitable composition to any of the accessible mucosal membranous tissue.

The term "nutrient" as used herein, refers to any compound that nourishes or promotes growth and development in humans and animals and includes compositions such as vitamins, proteins, amino acids, minerals, fats, carbohydrates and the like. In this invention, such compositions may be administered orally, by hyperalimentation, or any other acceptable means.

The following drug categories contain the pharmaceutical compounds which can be usefully delivered by means of the compositions and methods of the present invention.

Cardiovascular drugs; autacoids; drugs affecting renal function and electrolyte metabolism; drugs which act on synaptic and neuroeffector junctional sites; psychotropic drugs including anxiolytics, antidepressants, and antipsychotics; drugs affecting uterine motility; locally active drugs; antiparasitic agents; antimicrobials; xanthines; antineoplastics; immunosuppressants; immunomodulators; hormones and hormone antagonists; lipid-soluble and water-soluble vitamins and combinations thereof; also volatile anesthetics; local anesthetics; hypnotics and sedatives, that are medically appropriate and hypnotic; and sedatives such as chloral derivatives; e.g., chloral hydrate; and drugs useful for diagnostic purposes.

The drugs that are useful within the compositions of the present invention include, without limitation, any compounds that are physiologically active such as drugs which act at synaptic and neuroeffector junctional sites; cardiovascular drugs; autocoids; drugs affecting renal function and electrolyte metabolism; psychotropic drugs including anxiolytics, antidepressants, and antipsychotics; drugs affecting uterine motility; locally active drugs; antiparasitic agents; antimicrobials; xanthines; antineoplastics; immunosuppressants; immunomodulators; hormones and hormone antagonists; lipid-soluble and water-soluble vitamins and combinations thereof.

The following drug categories are examples of medically useful compounds which can be usefully delivered by means for the compositions and methods of the present invention. While the following general drug categories and examples are directed to individual drugs, the drugs from any categories can be combined in the compositions of the present invention:

Volatile anesthetics: methyl ether, divinyl ether, fluoroxene, halothane, etomidate, anesthetic steroids, enfluane, inoflurane, and combinations thereof. Intravenous anesthetics such as sodium pentothol, sodium amytal.

Local anesthetics such as cocaine, procaine, lidocaine, bupivacaine, chloroprocaine, dibucaine, etidocaine, mepivacaine, prilocaine, cyclomethycaine, hexylcaine and pramoxine, and combinations thereof.

Hypnotics and sedatives such as chloral derivaties, e.g., chloral hydrate; the benzodiazepines, such as diazepam and chlorazepate; and drugs in other subclasses of this category such a loxapine, molindone, chloral hydrate, chloral betaine, ethchlorvynol, ethinamate, glutethimide, meprobamate, methaqualone, mathyprylon, paraldehyde, and tricloros; and combinations thereof.

Drugs used to treat psychiatric disorders such as the following: the butyrophenones, such as droperidol and haloperidol; the phenothiazines, such as chlorpromazine, the thixanthines, such as chlorprothixene as well as the antidepressants including tricyclics such as imipramine, desipramine, amitriptyline, nortriptyline, doxepin, protriptyline, and isocarboxazid; MAO inhibitors such as phenelzine and tranylcypromine; and combinations thereof.

Drugs used to treat anxiety disorders such as meprobamate, benzodiazepam compositions such as diazepam, chloradiazepoxide, oxazepam, chlorazapate and combinations thereof.

Drugs used to treat epileptic disorders such as phenytoin, mephenytoin, ethotoin, primidone, carbamazepine, ethosuximide, methsuximide, phensuximide, valproic acid, valproates, trimethadione, paramethadione, and phenacelmide, and combinations thereof.

Narcotic analgesics and antagonists such as morphine and other opium alkaloids, meperidine and congeners, methadone and congeners, narcotic antagonists such as naxalone and feasible combinations thereof. Also drugs useful as centrally acting muscle relaxants such a L-dopa, amantadine and combinations thereof.

Nonsteroidal anti-flammatory drugs including the salicylates and salicylate-like compositions; the vicinal and geminal organophosphonates; the gentisates, such as phenylbutasone, indomethacin, oxyphenbutazone, antipyrine, aminopyrine, apazone, acetaminophen, phenacetin, sulindac, flufenamates, mefenamates, tojmetin, ibuprofen, naproxen, fenoprofen, flurbiprofen, ketoprofen, colchicine and allopurinol; and mucolytics, such as acetylcysteine. Also sympathomimetic drugs selected from the catecholamines, such as epinephrine and isoproterenol; amphetamines, including methamphetamine, hydroxyamphetamine; ephedrine, mephentermine, metaraminol, phenylephrine, methoxamine, methoxyphenamine, metaproterenol, and terbutaline; and combinations thereof; adrenergic blocking agents, including the alpha-adrenergic blockers, such as phenoxybanzamine; beta-adrenergic blockers, such as propranolol; adrenergic neuron blockers, such as guanethidine and bretylium; and useful combinations thereof.

Also useful are compositions including natural and synthetic histamines, 5-hydroxytryptamine, and combinations thereof. Similarly, autocoid antagonists such as those selected from histamine ($H_2$)-receptor blockers, and 5-hydroxytryptamine antagonists, such as the ergot alkaloids, lysergic acid derivatives, and cyproheptadine, as well as compatible combinations.

Also diuretic compounds such as acetazolamide; the benzothiadiazides, such as hydrochlorothiazide and methychlorothiazide; and the agents ethocrynic acid, furosemide, bumetanide, muzolimine, spironolactone, triamterene, amiloride, ticrynafen and indacrynic acid, and combinations thereof.

Also antimicrobial, antiviral and antiobiotic compounds including erythromycins; sulfonamides; caphalosporins; aminoglycosides; tetracyclines, rifamycins; lincomycins; polymyxins; chloramphenicol;

isoniazid; pyazinamide; cycloserine; viomycin, clindamycin; spectinomycin; vancomycin; nystatin; amphoteracin D, griseofulvin, amantadine; methisazone; vidarabine; idoxuridine; acyclovir, interferon; and feasible combinations thereof.

Antineoplastic agents, including compounds from various classes, such as ethyleneimine derivatives, alkyl sulfonates, mitrosources, triazenes, folic acid analogs, pyrimidine analogs, purine analogs, vinca alkaloids, 1-asparaginase, dactinomycin, daunorubicin, doxorubicin, bleomycin, mithramycin, mitomycin, platinum coordination complexes, substituted ureas, procarbazine, mitotane, tamoxifen, and combinations thereof.

Hormones such as growth hormone, prolactin, placental lactogen, luteinizing hormone, follicle stimulating hormone, thryotropin, chorionic gonabotropin, chorionic thyrotropin, corticotropin, alpha and beta-melanocyte stimulating hormones, beta-and gamma-lipotropins, endorphins, enkephalins, estrogens, projestins, adrogens, anabolic steroids, glucocorticoids, glucocorticoid derivatives, insulin, glucagon, tarathyroid hormone, thyroid hormone, protaglandins, calcitriol and calcitonin. Also immunomodulators, such as cyclosporines and proteins known to have such effects.

## THE FINISHED PRODUCT

Finished products of this invention are comprised of microencapsulated coacervate-based matrix particles of the active component(s) in any appropriate pharmaceutical dosage form including emulsions, suspensions, microemulsions, powders, microsuspensions, foams, liquids, gels, tablets, capsules or any other pharmaceutically acceptable dosage form or combinations thereof as preferred by the formulator.

The active component may be any drug, or combinations thereof, or any biological or combinations thereof, or combinations of drug(s) and biological(s) or the active component may be antigenic compound as present in vaccines or marker compositions as used for diagnostic and immunoassay procedures. It is understood that the microencapsulated particles also may contain compounds such as preservatives, antioxidants, electrolytes and the like, to preserve or enhance the activity of the incorporated component(s), as preferred by the formulator.

In this method, each microparticle is comprised of a matrix particle containing the active compound(s) preferably enveloped by a single encapsulating film or if desired, by multiple films that are structured ranging from semi-liquid or gel-like to semi-solid to solid. The matrix particles are preferably comprised of coacervate phase water, equilibrium phase water or mixtures and from 1% to about 99%, preferably less than 25%, by total weight of the particles, of the egg components, surfactants and other components as are used to solubilize the drug and form the two phase coacervate composition. In one embodiment of the method, the surface of the particle is in a different physical-chemical state than the interior of the particle containing the physiologically-active compound thereby imparting the particle surface with a film-like characteristic. Finished products of this method can be prompt or sustained release particles or combinations thereof. Particles of finished or semi-finished products can be prepared to sizes ranging from 0.125 microns or less to 100 microns or more in any single dimension as preferred. According to the methods of the present invention, microencapsulated coacervate-based matrix particles are prepared to sizes that are physiologically appropriate and, in the case of drug and nutrient formulations particles, are of a size that favors effective absorption by the body. Finished products can include the same or different size particles.

Finished or semi-finished particles are structured, if desired, by using a non-toxic alcohol such as ethyl alcohol, 100-200 proof.

As an option, finished or semi-finished particles can be treated by physical processes such as heat to structure the surface of the coacervate-based matrix particles and/or its encapsulating film.

For example, the particles, with or without an encapsulating film, may be structured by heating from about 40°C to about 100°C for from about 5 seconds to about 2 minutes to achieve the desired degree of structure. The degree of heat used is governed by sensitivity of the active component to heat and the desired degree of particle and/or particle film structure.

As another option, the particles, with or without an encapsulating film, may be structured with chemicals other than alcohol such as a crosslinking agent, for example, an aldehyde, to increase the structure of the composition. For example, about 1% w/v of gluteraldehyde can be mixed into the composition. When the desired degree of structure has been achieved, the microparticles are washed with distilled water by conventional methods until all traces of the crosslinking agents have been removed. The resulting product then can be dried by any of the conventional means and either stored or dispensed in any appropriate dosage form.

The parameters that determine the pH of the formulations of this method include physiological compatibility, mode of product administration and sensitivity of the active component to pH with respect to stability, activity, and the like. In general, a pH of about 7.4 is preferred. However, as required, the pH of the formulation may vary from 7.4 provided the finished product is physiologically acceptable.

As appropriate, the finished products, based on the method of this invention, can be administered to humans and animals by means of ophthalmic, oral, topical, transdermal, parenteral, tissue absorptive, and inhalation methods or combinations thereof as medically appropriate. It is preferred that devices based on ultrasound or other devices that facilitate passage of drug compounds through the skin be used in conjunction with the application of the topical and transdermal products of this method. Devices which facilitate absorption of the drug(s) by producing a cavitation effect on the skin are preferable.

The object of the present invention is to provide delivery systems, one application of which includes methods and compositions directed to the delivery of water-insoluble drugs, lipid-soluble drugs, water-soluble drugs, and drugs which are preferentially lipid-soluble and combinations thereof.

It is another object to provide microencapsulated drugs which can be prepared for delivery in any of the conventional dosage forms and introduced into or on the surface of the body.

Another object of the present invention is to provide microencapsulated, preferably coacervate-based matrix particles which comprise a red blood cell substitute and a method to produce the composition and related products.

Another object of the present invention is to provide a resuscitation fluid based on the use of the red blood cell substitutes of the present invention.

Another object is to provide an oxygen carrying organ and tissue perfusion fluid based on the use of the red blood cell substitute contained in preferably coacervate-based matrix particles made in accordance with the present invention.

Another object is to provide an oxygen carrying composition for in vitro cell and tissue culture.

According to one embodiment, the present invention provides a drug delivery composition and a method of making the composition wherein one or more physiologically active compounds or medically useful material is dissolved or dispersed in a liquid solvent or carrier prior to incorporation in coacervate-based matrix particles.

Further, the present invention provides a delivery composition and a method of manufacture wherein physiologically active or medically useful compounds are stabilized and improved by incorporation in microencapsulated, preferably coacervate-based matrix particles prepared in accordance with the present invention.

The above objects and the advantages of the present invention will become apparent from the following detailed description of the preferred embodiments.


## BRIEF DESCRIPTION OF THE DRAWINGS


Fig. 1 is a graph illustrating the immediate glucose lowering effects of two compositions prepared according to the present invention, when administered orally to rats, in comparison to prior art insulin when taken orally; and

Fig. 2 is a graph illustrating the effects of the oral administration of insulin encapsulated in accordance with the present invention when administered to rats that have been pretreated to prevent the natural production of insulin, in comparison to admininstration of insulin unencapsulated administered orally and by an injection.


## DETAILED DESCRIPTION OF THE INVENTION


The following are specific embodiments and methods of the disclosed delivery systems and applications thereof. Also included are method options available to the formulator. Semi-finished and finished products of this method can be formulated in any dosage forms and by any modes of administration.

According to the methods and compositions of the present invention, the quantity of the active component incorporated in the compositions, i.e., drug(s), biological(s), nutrients, and the like, may vary according to the requirements of the formulator. In the following detailed description, it is to be understood that unless otherwise specified, the term "required quantity" or "desired quantity" of active components is used to mean that amount of said component(s) as will give either the desired or the standard dose in the finished product. The Physician's Desk Reference (Medical Economics, Oradell, N.J., 1989) Goodman, L. and Gilman, A., The Pharmacological Basis of Therapeutics, MacMillan, 7th Edition and the AMA Drug Evaluation, 5th Edition, 1986 (American Medical Association) may be used as guides for standard and other appropriate doses and are hereby incorporated in this disclosure by reference. In general, the required starting quantity will be about equal to the amount of drug present in the finished product. In other preparations, depending on the election of the formulator, the starting required quantity of drug or other component may be from about 0.5% to about 75% more than will be present in the finished product. Alternatively, at the option of the formulator, the required amount of the active component may be any fraction of the standard dose but especially as delivery formulations of this invention improve the bioavilability, stability and other features of the incorporated component(s).

In the instance of the disclosed resuscitation fluid, the "red cell" component may contain about 5 to about 50 weight percent of stroma-free hemoglobin; about 14 percent by weight is preferred. In the instance of a perfusion fluid, the hemoglobin component in the "red cells" of the perfusion fluid may range from about 5 to about 50 weight percent; about 14 weight percent is preferred. In the instance of the disclosed oxygen carrying fluid used for angioplastic procedures, the stroma-free hemoglobin component can range from about 2 to about 15 weight percent; about 7 weight percent is preferred.

## ORAL AND PARENTERAL DRUG DELIVERY FORMULATIONS

In the practice of this invention which relates to drug delivery, base compounds or the salts thereof may be used as starting materials. Parent drug compositions are preferred. In specific instances, some drugs require solubilization, melting, conversion to the base drug or other processing before being formulated according to the method of this invention.

The first steps of the disclosed method produce a useful basic semi-finished composition including microencapsulated coacervate-based particles containing the drug component suitable for oral administration. In preferred practice, these preparations are processed further to improve the semi-finished product but more particularly to produce the desired degree of surface film and/or matrix surface structuring of the microparticles referred to above. The disclosed method provides for useful processing variations to produce oral delivery formulations. The following are descriptions of the methods to prepare oral drug compositions:

The starting quantity of the active component may be equal to or greater than the finished product dose depending upon the characteristics of the formulating process. In instances wherein the product is subjected to spray drying for example, some loss of drug occurs. In such instances, the starting quantity of the drug may be increased according to the requirements of the formulator.

(1) Disperse one or more lipid-soluble or water-insoluble drug(s) or other similar medically useful components in a mixture comprised of the yolk and white of one egg, preferably filtered using filters having openings of 5 microns or less. As an option, the quantity of the egg yolk and egg white may be greater or lesser than the quantity contained in a single egg depending upon the requirements of the formulator. The quantity of drug(s) used should be that amount as will give the desired unit dose in the finished product.

As required, the amount of egg whte used in formulations of this method can range from about 1/10 of the egg white of one egg to the white of about three or more eggs. As an option, from about 0.1% to about 4% w/v or more of any proteinaceous compound, without limit, as exemplified by albumin, coacervated albumin, and lyophilized albumin, singly or in any combination may be used in place of or in conjunction with egg white.

The quantity of egg yolk used in this method can range from about 1/10 of the egg yolk of one egg to the yolks of about 3 eggs or more depending upon the formulator's requirements. As an option, a phospholipid such as lecithin, egg phosphatides, coacervated egg lecithin, lyophilized lecithin, either singly or in any combination, may be added and used in conjuction with, or as a substitute for, the egg yolk. The amount of phospholipid added or substituted can range from about 0.50% to about 4% w/v or more. If a substitute is used for egg white, egg yolk or derivatives thereof should be present in the formulation in forming the coacervate composition. Similarly, if a substitute is used for egg yolk, egg white or a derivative thereof should be present in the formulation in forming the coacervate composition.

Next, (2) preferably, a lipid compound with solubilizing and surfactant properties is mixed into the product of step (1). The lipid compound may be in the form of an oil, a fat, an essential fatty acid, or a fat-soluble surfactant. The preferred lipid compound is in the form of a mixture of purified vegetable mono- and diglycerides, particularly glycol mono-oleate or a combination of glycerol mono-oleate and glycerol monostearate. The lipid compound is added in an amount of about 1% to about 2% of the total weight of the finished product.

Next, (3), process the composition for approximately 2 to about 20 minutes in a colloid mill, microfluidizer or other similar equipment to form coacervate-based matrix particles of any desired particle size. Colloid mill processing for about 10 minutes is generally sufficient. During this processing step (4), add a structuring or coacervating agent dropwise, preferably any suitable alcohol, such as pure grain alcohol or any non-toxic alcohol, preferably at an aqueous concentration of 100 proof or more, to the composition. The amount of alcohol added during the colloid mill processing step depends upon the degree of particle matrix structuring and, more particularly, of particle surface film structuring desired by the formulator.

The quantity of alcohol used in this method can range from about 20 mls to about 200 mls depending on the formulator's requirements and its concentration. About 50 mls of pure grain alcohol produces a useful degree of structure. The structure of the microparticles resulting from step (4) can range from semi-liquid or gel to semi-solid or solid as required depending upon the qualtity and concentration of alcohol or other structuring agent used during the process of step (4).

If desired, a sterol such as cholesterol in an amount ranging from about 0.1% to about 5% or more w/v may be added to the preparation during the colloid mill processing step to enhance the structure of the surface of the microencapsulated particles.

(5) The product of step (4) is washed thoroughly with sterile water and then dried by any of the conventional techniques and/or lyophilized as desired. As required, the product may be filtered to produce compositions having any desired particle size or mixtures of particle sizes. The product comprises an oral composition containing microencapsulated particles or coacervate phase water, equilibrium phase water or a combination thereof containing the drug or other active components.

A useful variation of the method described above also includes the use of mono-, di-, or triglycerides in combination with egg yolk and/or egg white components. In this instance, the desired quantity of the drug component(s) or other medically useful compound(s) is (1) dispersed in a medium-chain glyceride ($C_6$-$C_{12}$ fatty acid chains) in about that ratio as will give the desired unit dose in the finished product. Depending on the drug to be dispersed, its stability and solubility, the proportion of drug to glyceride will vary. The quantity of drug can range from about five times more, by weight, or less, than the quantity of glyceride. For example, approximately 4 grams of erythromycin may be dispersed in about 1 gram of a medium chain glyceride, using a colloid mill to facilitate dispersion. The quantity of the di-glyceride component may be increased or decreased depending upon the solubility of the drug or the requirements of the formulator.

Next, (2), the resulting composition is mixed into the yolk, preferably filtered, of one egg. As an option, about 1% to about 2% w/v of a phospholipid such as coacervated egg lecithin may be added into the preparation or used in place of the preferred egg yolk. Another option comprises the addition of about 1% to 2% w/v of albumin or egg white, preferably filtered egg white, to the composition. (3) The resulting product then is processed in a colloid mill for approximately 5 to 10 minutes during which time about 25 to 30 mls of ethyl alcohol are added dropwise. If desired, the product of step (3) may be filtered to cbtain microencapsulated drug particles of the required size(s) or combination of sizes. The product of step (3) comprises a composition which may be administered in any suitable oral dosage form or lyophilized for use in subsequent formulations.

This method also provides formulations for the oral delivery of water-soluble drugs. The formulation process is as follows: Disperse that qualtity of the drug in the egg white, preferably filtered, of one egg. The quantity of drug is that amount that will yield the desired dosage in the finished product. As an option, the qualtity of egg white may be increased or decreased as required by the formulator. If desired, a suitable dispersing agent such as a phospholipid, i.e., egg yolk, lecithin, preferably coacervated lecithin, may be added to the product of step (1) in an amount ranging from about 0.5% w/v to about 10% w/v or more. As an option, any suitable water-soluble proteinaceous material with surfactant properties, such as albumin, coacervated albumin, glycoproteins, gelatins and the like, or combinations thereof, may be added to the product of step (1) in amounts from about 0.5% to about 5% or more, or used in place of egg white. Another option includes the addition of antioxidant such as vitamin C or tocopherols, i.e., vitamin E, and the like, or any acceptable combination of antioxidants. The quantity of antioxidants used in this method can range from trace amounts to about 5% w/v.

Next, (2), the composition is processed in a colloid mill for approximately 5 to 10 minutes, adding dropwise about 25 mls of a structuring agent, such as alcohol, preferably pure grain alcohol or other alcohol preferably having a concentration of 150 proof or more. As an option, a sterol such as cholesterol, in an amount ranging from about 0.1% w/v to about 5% w/v, may also be added during step (2) to increase the degree of film structure. (3) It is preferred that a preservative such as methyl paraben, or propyl paraben, or combinations thereof, is added during the colloid mill processing step (2) in amounts ranging from trace quantities to about 10 mls. Following step (3) the finished product may be lyophilized or stored, preferably under refrigeration at about 4 to about 10° C or administered in any appropriate oral dosage form.

As desired, step (2) of this method may be repreated as often as necessary to produce microparticles with the desired sustained release characteristics.

The microencapsulated drug particles produced by the methods described above may be regarded as semi-finished products. They nonetheless comprise useful oral drug delivery compositions. As desired, the microparticles of these formulations may be filtered to remove all particles exceeding any given size. As an option, the semi-finished products of these methods may be used as staring materials to prepare the topical, opthalmic, tissue absorptive, transdermal or inhalation compositions of this invention, described in more detail hereinafter.

In the practice of this invention, it is preferred that the semi-finished products of the methods described above be processed in the following manner to produce encapsulated drug compositions that comprise finished compositions.

(1) Mix the semi-finished product (microencapsulated coacervate-based matrix particles having a solubilized drug in the coacervate phase water and/or equilibrium phase water) prepared by one or more of the methods described above, preferably lyophilized microencapsulated particles, in the white, preferably filtered, of one egg. Use the microencapsulated drug particles in that quantity as will give the desired per unit dose of drug in the finished product.

As an option, about 0.1% to about 4% w/v of a proteinaceous compound such as albumin, coacervated albumin and the like may be used in conjuction with or in place of egg white. The quantities of the components used in step (1) may be increased or decreased in accordance with the formulator's requirements. (2) Next, process the product in a Tissuemizer or other colloid mill for about 5 to about 10 minutes, adding approximately 25 to 50 mls of a structuring agent such as an alcohol, preferably grain alcohol at a concentration of at least 180 proof. As an option, a sterol such as cholesterol, in an amount ranging from about 0.1% w/v to about 5% w/v, may also be added during step (2) to increase the degree of film structure. While not preferred, the process of step (2) may be reversed; i.e, the product may be mixed into from 25 to 50 mls of grain alcohol, 180 proof, and processed in a colloid mill, as described above. (3) The product of step (2) is washed thoroughly with sterile water and dryied using any conventional drying method. When spray drying is used, the particles are first dispersed in an appropriate vehicle, such as distilled water, alcohol, and the like, before such processing. Following the drying step, the microencapsulated particles containing the incorporated drug, nutrients or other useful physiologically-active components, in the desired formulation, comprise the finished product.

The compositions produced by this method may be characterized as follows: the size of the particles produced by these methods range from less than 0.125 micron to 100 microns or more. Preferred size is any size that facilitates absorption of the composition or that meets the preference of the formulator. Finished products may be comprised of a single size particle or mixtures of particle sizes. Microfluidizing the product and use of filter steps using appropriately sized filter pores produce microparticles of the preferred size (s) according to this method.

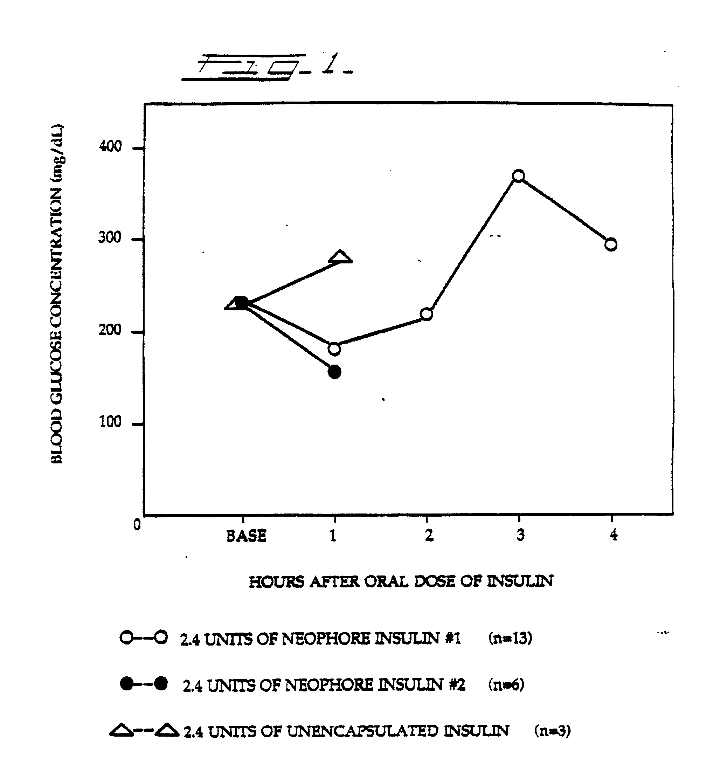

Referring now to Fig. 1., 2.4 units of insulin were encapsulated within a combination of egg white and yolk as described:

| NEOPHORE INSULIN #1 | |
| --- | --- |
| Insulin | 0.1 g |
| Coacervated egg lecithin | 5 drops |
| Coacervated egg albumin | 100 ml |
| Methyl Paraben | 0.018 g = 18 mg |
| Propyl Paraben | 0.002 g = 2 mg |
| Grain Alcohol 190 proof | 70 ml |

The composition was prepared as follows:

#1 Add 0.1 g of bovine pancreas insulin into 100 ml of coacervated egg albumin, while blending at a low speed in a sigma blender for 3 minutes.

#2 Add 5 drops of coacervated egg lecithin (drop by drop) into #1 while blending at a low speed in a sigma blender for 3 minutes.

#3 Continue blending and add 1 mg of stock solution (methyl paraben, 1.8 g; propyl paraben, 0.2 g; grain alcohol to 100 ml); and 69 ml of 190 proof ethyl alcohol into #2.

16

#4 Place #3 in freezer for three hours.

#5 Lyophilize #4.

A uniform lyophilized powder reusulted, with no crystals, having a weight of 12.96 grams containing 7.7 milligrams of insulin per gram.

| NEOPHORE INSULIN #2 | |
|---|---|
| Insulin | 0.1 g |
| Coacervated egg lecithin | 3 drops |
| Coacervated egg albumin | 50 ml |
| Methyl Paraben | 0.009 g = 9 mg |
| Propyl Paraben | 0.001 g = 1 mg |
| Grain Alcohol 190 proof | 50 ml |

The composition was prepared as follows:

#1 Add 0.1 g of bovine pancreas insulin into 50 ml of coacervated egg albumin. Mix well with glass rod.

#2 Add 3 drops of coacervated egg lecithin into #1 (using a syringe without the neddle). Mix well with glass rod. Blend the suspension in a Tissumizer for 3 minutes.

#3 Add 0.5 ml of stock solution (methyl paraben 1.8 g; propyl paraben, 0.2 g; grain alcohol, to 100 ml) and 49.5 ml of grain alcohol into #3, while tissumizing for 3 minutes on low speed.

#4 Place #3 in freezer (overnight).

#5 Lyophilize #4.

#6 Sieve and mix #5 using a #20 sieve, mortar and pestle.

#7 Mix #6 using a spatula.

Weight of powder = 6.5 g having a particle size such that 100% of the particles pass US #20 screen.

Encapsulated insulin #1 was administered orally to 13 rats (n = 13), and encapsulated insulin #2 was administered to 6 rats (n = 6) by incubating the rats and forcing the insulin through a catheter in fluid communication with the rats' stomach. The unencapsulated insulin was insulin, normally adminsitered by injection, and was orally administered to 3 rats (n = 3) also by forcing the insulin through a catheter in fluid communication with the rats' stomach. The glucose levels in the rats' blood were measured at various times after administration, an average level calculated, and glucose level vs. time, plotted in Fig. 1. As shown, the encapsulated insulin compositions of the present invention (Neophore Insulin #1 and Neophore Insulin #2) rapidly decreased the glucose levels in the rats' blood streams more than 30% within the first hour, whereas the unencapsulated insulin had no effect, allowing the glucose levels in the rats' blood streams to rise about 15% within the first hour. Only Neophore insulin #2 was measured beyond the first hour and the glucose level substantially rose between hours 2 and 3 but dropped again by hour four. Increased dosages of encapsulated insulin, or insulin in a time release form in accordance with the film hardening technique of the present invention can provide the desired release of insulin with time.

As shown in Fig. 2, 2.4 units of unencapsulated insulin administered by injection; 2.4 units of unencapsulated insulin adminstered orally; and 2.4 units of orally administered insulin encapsulated in accordance with the present invention were given to 10 rats (n = 10). The rats were pretreated with drugs to prevent the natural bodily secretion of insulin within the rats. The insulin administered orally was forced through catheters in fluid communication with the rats' stomachs. The glucose levels in the rats' blood were measured at various times after administration, an average level calculated, and glucose level vs. time plotted in Fig. 2. As shown in Fig. 2, the injected insulin caused a rapid decrease (within about 10 minutes) in blood glucose levels to an extent that could cause hypoglycemia in mammals. The unencapsulated insulin administered orally prevented a rapid further rise in glucose levels within the first hour or hour and a half, but the glucose level began rising after that time. The encapsulated insulin administered orally in accordance with the present invention required about 25 minutes to begin glucose level reduction, but caused an even gradual leveling of the glucose level back to the original level of about 120 mg/dl for the particular rats tested. Although the rats tested with the orally administered insulin encapsulated in accordance with the present invention had initially higher glucose levels than the rats that were injected with unencapsulated insulin, the glucose curves were approximately the same, with the insulin composition of the present invention causing a steadier, more even decrease in glucose levels thereby preventing hypoglycemia and other possible problems resulting from too rapid a decrease in blood glucose.

If desired, time release particles containing insulin in accordance with the present invention can be combined with insulin-containing particles having no surface hardening to provide more rapid insulin release.

In addition, the methods of this invention produce a red blood cell substitute, and oxygen carrying compositions such as a resuscitation fluid, perfusion fluids and cell culture media based thereon.

The film encapsulating the microparticles produced by the method of the present invention may be a single coacervate-based layer of any desired thickness, or if preferred, the film may be multilayered. The encapsulating process of this method can be repeated as often as required to produce particles with the described sustained or prolonged drug release characteristics.

Finished products may be comprised of microparticles with a single drug release rate or a mixture of release rates as required. The degree of particle matrix surface and/or encapsulating film structure can be varied in a range from semi-liquid or gel-like to semi-solid to virtually solid, as required. According to this method, the quantity of alcohol or other chemical structuring agent and, as preferred, the quantity of cholesterol used in the manufacturing steps of this method are among the primary structuring agents. In addition, the heat generated by some of the processing steps, e.g, colloid mill processing, may also exert a structuring effect on the matrix particle surface and/or the applied films.

The microencapsulated drug particles may be lyophilized and/or dispensed in the required per unit dose in any suitable oral pharmaceutical vehicle and in any pharmaceutically acceptable dosage form, e.g., emulsions, microemulsions, suspensions, tablets, wafers, capsules and the like. Alternatively, as preferred, the semi-finished and finished products produced by the methods described above may be used as starting components for the topical tissue absorptive, transdermal, opthalmic and inhalation drug formulations of this invention, described in more detail hereinafter.

To prepare the microencapsulated parenteral drug formulations of this invention, the components thereof preferably are used in quantitites within the following ranges: the drug or other active component: that amount as will yield the desired per unit dose in the finished or semi-finished product; egg yolk: preferably filtered through filter pores from about 2 to about 5 microns, from about 2 ml to about 150 ml; egg white: preferably filtered through filter pores of about 2 to about 5 microns, from about 25 to about 75 ml; as an option, add sodium alginate: from about 0.2 gram to about 15 grams; colloid rich phase and/or colloid poor (equilibrium water) phase of the described coacervate system: coacervate (colloid rich) phase from about 50 to about 350 mls; equilibrium (colloid poor) phase: from about 30 to about 200 mls; methyl paraben: from about 0.01 gram to about 1.0 gram; propyl paraben: from about 0.001 gram to about 0.085 gram. In the preferred practice of this invention, the weight ratio of methyl paraben to propyl paraben is about 1 to about 10; grain alcohol, preferably 180 or more proof: from about 100 mls to about 300 mls. The formulator may adjust these quantities as required.

The homogenizing steps are an important feature of the process of the present invention. A Tissuemizer or a Microfluidizer or both or any similar apparatus may be used in the homogenizing steps as desired.

The following are descriptions of preferred methods useful to produce parenteral delivery compositions for drugs, bioactive proteins and other medically useful compositions.

(1) Dissolve about 0.1 gram of methyl paraben in about 20 ml of distilled water heated to about 80° C; (2) dissolve about 0.1 gram propyl paraben in about 100 mls of distilled water heated to about 100° C. Concentrations of the parabens, used as preservatives, may be increased or decreased as preferred. Allow both preparations to come to room temperature. Next, (3) add the desired quantity of the drug component in small increments to about 50 ml of egg white; homogenize the product using a Tissuemizer for about 30 minutes. Next, (4) add from about 1.5 to about 5 mls of the product of step (1) and about 0.5 to about 2 mls of the product of step (2) into the product of step (3). It is preferred that about 2.2 mls of methyl paraben solution and about 1 ml of propyl paraben solution be used. Next, (5) homogenize the product of step (4) using a Tissuemizer for about 10 minutes while adding from about 50 to about 100 mls of alcohol. Discard any foam that develops during this step. As an option, the pH of the product may be adjusted to about 7.4 using 1N HCl or NaOH as required. The composition comprises microencapsulated coacervate-based matrix particles containing the incorporated active component(s) useful for parenteral formulations.

A modification of the method given immediately above also produces a useful parenteral product. In this embodiment, the following steps are used. (1) dissolve about 0.01 gram of methyl paraben in about 20 ml of distilled water heated to about 80° C; (2) dissolve about 0.001 gram propyl paraben in about 100 mls of distilled water heated to about 100° C. Concentrations of the parabens may be increased or decreased as desired. Allow both preparations to come to room temperature. Next, (3) add the described quantity of the drug component in small increments to about 60 ml of egg white; homogenize the product using a Tisuemizer for about 30 minutes. Next, (4) add from about 1.5 to about 5 mls of the product of step (1) and about 0.5 to 2 mls of the product of step (2) into the product of step (3). It is preferred that about 2.2 mls of methyl paraben solution and about 1 ml of propyl paraben solution be used while adding about 50-100 mls of alcohol Next, (4) homogenize the product of step (4) using a Tissuemizer for about 10 minutes. Discard any foam that develops during this step. The pH of the product may be adjusted to about 7.4 using 1N HCl or NaOH as required. The composition comprises an optional parenteral product or the product can be further processed as follows. Next, (5) add from about 5 to about 7.5 ml of egg yolk into the product of step (4); homogenize the resulting composition using a Tissuemizer for about 15 minutes. As desired, adjust the pH of the product to about 7.4 using 1N HCl or NaOH as necessary. The composition comprises microencapsulated coacervate-based matrix particles containing the active component useful for parenteral formulations. As an option, the product may be filtered using filter pores of the desired size to produce particles of any size.

Another useful variation of the method for preparing parenteral drug compositions comprises the following steps: (1) Mix the required amount of drug into about 45 ml of the coacervate phase of a two phase coacervate composition prepared using about 80 grams of propylene glycol E 8000; about 200 grams of dextrin having a molecular weight about 10,000 to about 40,000; a molecular weight of about 19,500 is preferred; and about 800 mls of distilled water. Process the composition in a Tissuemizer for about 10 to about 15 minutes; (2) Disperse about 0.1 gram of methyl paraben and about 0.01 gram of propyl paraben in about 60 ml of filtered egg white and process the product until a uniform composition is obtained. Any acceptable grinding or mixing process may be used to facilitate the dispersion referred to above. Next, (3) add the product of step (2) preferably in small increments into the product of step (1) while homogenizing the composition with a Tissuemizer or other apparatus until a uniform suspension is produced. Next, (4) add about 100 mls of alcohol preferably in small increments to the composition of step (3) while processing the product in a Tissuemizer until a uniform suspension is obtained. On completion of step (4) add about 12 ml of filtered egg yolk into the product of step (4) and homogenize the product using a Tissuemizer for about 15 minutes. Adjust the pH of the composition to about 7.4 by using 1N HCl or NaOH as necessary. The product comprises microencapsulated coacervate-based matrix particles containing the drug suitable for parenteral use.

Another variation of the method to prepare a parenteral drug formulation comprises the following: (1) dissolve about 2 grams of ethyl cellulose in about 50 mls of grain alcohol, preferably about 190 proof. Next, (2) dissolve the required amount of water-soluble drug in about 20 mls of distilled water. (3) Add the product of step (2) dropwise into about 5 grams of egg yolk; process the composition using a Tissuemizer during the addition step. Next, (4) add the composition of step (3) dropwise into about 6 grams of egg white while continuing to homogenize the product using a Tissuemizer for about 10 minutes. Next, (5) add the product of step (4) to the product of step (1) and homogenize the composition using a Tissuemizer until a uniform suspension is produced. (6) Adjust the pH of the composition to about 7.4 using HCl or NaOH as required. The product comprises microencapsulated coacervate-based matrix particles containing the drug within the particles in solution as a parenteral drug formulation.

Yet another variation of the method comprises the following: (1) dissolve and mix about 2 grams of ethyl cellulose in about 50 ml of grain alcohol, preferably 190 proof. Next, (2) dissolve the required amount of drug in the minimum quantity of grain alcohol necessary to dissolve the drug by adding increments of about 5 mls of grain alcohol until the drug component is completely dissolved. (3) Mix the products of steps (1) and (2) using a Tissuemizer for about 10 minutes. Next, (4) add about 15 mls of egg white to the product of (3) and process the resulting product with a Tissuemizer for about 10 minutes. (5) Adjust the pH to about 7.4 using 1N HCl or NaOH as necessary. (6) Next, the product is subjected to lyophilization. (7) The lyophilized product is added to a normal saline solution to produce the finished parenteral composition.

To prepare a sustained release form of parenteral formulations, (1) disperse the desired quantity of the finished microencapsulated product prepared from any of the previously described parenteral or oral formulations in the white of one egg or as an option, in about 60 mls of albumin or in about 60 mls of any suitable combination of egg albumin and egg white. The quantity of the albumin or the preferred egg white may be increased or decreased in such combinations as desired by the formulator. (2) Process the product of step (1) in a Tissuemizer for about 10 minutes. Next, (3) during this homogenizing step, add about 200 mls of grain alcohol, about 190 proof, dropwise to the composition. (4) About about .04 gram of methyl paraben and about 0.01 gram of propyl paraben in about 5 mls of polyethylene glycol added during the homogenizing step. At the end of step (4) the product may be lyophilized and stored until needed or (5) processed further as follows:

Disperse the quantity of the product of step (4) in that amount of physiological saline solution or other pharmaceutically acceptable vehicle as will give the required per unit dose in the finished product. (6) The dispersal step is followed by adjustment of the pH of the composition to about 7.4 using either HCl or NaOH as required, and if desired, (7) by adjustment of viscosity of the product to the formulator's requirements. (8) As an option, the compositions is filtered using appropriate size filters to remove all particles that do not conform to the desired micron or sub-micron size(s). (9) The product of step (8) can be processed as often as desired using steps (1) through (4) immediately above to produce sustained or prolonged release characteristics. The finished product of step (8) and step (9) can be administered in the form of an emulsion, microemulsion, suspension or microsuspension or any other form suitable for parenteral use.

## OPHTHALMIC FORMULATIONS

The starting materials for ophthalmic compositions may consist of the semi-finished or finished product-(s) including sustained release formulations produced by the methods to prepare the oral or parenteral products of the present invention. The finished oral drug compositions are preferred. Compositions suitable for ophthalmic administration are prepared using the following steps: (1) disperse the required quantity of the starting microencapsulated drug particles in about 10 mls of isotonic saline solution. The quantity of isotonic saline soltuion may be increased or decreased as required by the formulator. (2) Preferably, filter the composition to remove all encapsulated particles larger than 2 microns. (3) Adjust the pH of the composition to about 7.3 using either HCl or NaOH, as necessary. The finished product is administered to the eye in the form of droplets; the per unit dose may range from about 0.05% to about 10% of the drug component or more as required. In addition to drugs specific to conditions affecting the eye, the finished product may also be formulated to contain the required quantity of any drug or other medically useful composition that can enter the circulation by the ophthalmic route and exert a beneficial systemic effect elsewhere in the body.

## TISSUE ABSORPTIVE FORMULATIONS

The starting material(s) for the disclosed tissue absorptive compositions consist of semi-finished or finished microencapsulated particles comprised of one or more drugs prepared by any of the oral or parenteral methods of the present invention. Combinations of the products of these methods may be used as an option.

Finished microparticles produced by the oral method are preferred. The quantity of the starting microencapsulated drug is that quantity that will yield the desired unit dose of drug in the finished product. (1) Disperse the starting materials comprised of prompt or sustained release microencapsulated drug particles or any combination thereof in that quantity as will give the desired per unit dose in any pharmaceutically acceptable ointment, cream, gel or any coacervate-based gel. A coacervate-based gel is preferred, particularly from any albumin-lecithin, or a gelatin-lecithin or a two gelatin-based coacervate composition. (2) Dispersion of the starting material in the vehicle is accomplished using any conventional mixing or homogenizing methods. (3) The pH of the product is adjusted using 1N, HCl or NaOH as required to about 7.4 or, if desired, to the pH of the tissue involved. The finished product is applied directly to the target tissue. If desired, adjuvant compounds such as about 1% w/v urea, or about 1% w/v guanidine, or about 1% w/v carnitine or other similar compounds or combinations thereof may be added to the product during the dispersion step. Microencapsulated particles of drug 1 micron or less are preferred for the finished product of this method and are obtained by filtration of the staring material using filters of the required size. Particles larger than 1 micron also are useful in this method.

## INHALATION FORMULATIONS

To prepare a formulation useful to deliver drugs and other medically useful compositions by means of inhalation, the following method is used: (1) disperse that quantity of semi-finished or finished microencapsulated particles prepared by any of the oral or parenteral methods of this invention in physiological saline solution or other acceptable vehicle as will give a finished product with a unit dose ranging from about 0.5 ml to about 20 ml of a 5% w/v or, if preferred, a 10% w/v preparation. (2) The pH of the product should be adjusted after step (1) to about 7.4 using either HCl or NaOH as required. The particle size is an option to the formulator and can range from less than 0.125 micron to about 10 microns or larger. The finished product of this method may include prompt, and/or sustained release microparticles prepared by the methods described above and can be administered by nebulizer or by any metered dose inhalant device.

As an option, the following method is used: (1) disperse the particles described immediately above into about 20 mls of the coacervate phase of a coacervate system made of 80 grams polyethylene glycol 8000, 200 grams of dextrin, 19,500 weight average molecular weight and 800 grams distilled water. The resulting product can be administered by nebulizer or any metered dose inhalant device.

## TRANSDERMAL FORMULATIONS

Starting materials used in trandermal compositions of the present invention comprise semi-finished or finished microencapsulated particles of the active component(s) prepared by the method to produce microencapsulated drug products for oral or parenteral use or any combination thereof. As an option, adjuvant compounds such as about 1% w/v urea, about 1% w/v guanidine or other compounds and any combinations of such compounds known to facilitate drug transport may be added to the starting materials. (1) The required quantity of microencapsulated drug particles is mixed into any appropriate transdermal vehicle using any suitable mixing method to produce an even distribution of the drug particles throughout the vehicle. Acceptable vehicles include creams, ointments, gels, preferably coacervate-based gels and particularly coacervate gel formulations derived from albumin-lecithin or gelatin-lecithin or two gelatins-based coacervate compositions.

The quantity of microencapsulated drug particles used is that amount as will yield the desired per unit dose, as for example 1% w/v (10 mg/ml gram) 2% w/v (20 mg/ml) or more per unit application, or less as required. (2) It is preferred that the pH of the vehicle be adjusted to about the pH of the skin or as close to the pH of the skin as will maintain the stability of the drug. As an option, the formulator may adjust the composition to any pH appropriate to the effective transdermal transport of the active drug component(s). (3) The finished drug delivery composition containing the required dose may be applied directly to the skin or incorporated in an appropriate laminate, or a suitable medical tape or other acceptable device and applied to the skin. It is preferred that the finished product be used in conjunction with an ultra-sound or other sonic device that produces a cavitation effect on the area of the skin to which it is applied to enhance transport of the drug through the skin. As an option, transdermal formulations may be administered directly as topical medical preparation.

## FOAM FORMULATIONS

Foam formulations of medically useful compositions can be made by modifying the process used for making either any semi-finished or finished microencapsulated oral or parenteral compositions of this method. The modification includes the following steps: (1) Prior to the last colloid mill processing step in the methods to prepare semi-finished or finished oral or parenteral druG formulations, adjust the pH of the composition using HCl or NaOH so as to maintain stability of the active component(s). As an option, adjuvant compositions such as 1% w/v urea, 1% w/v guanidine and the like, and combinations thereof, in amounts which approximate a total of about 1% w/v may be added to facilitate drug absorption. (2) Next, filter the preparation to remove all particles larger than about 0.125 to about 0.5 microns and (3) bubble a physiologically-acceptable gas, such as nitrogen, into the preparation until the microemulsion is converted into a composition comprised of microencapsulated particles dispersed in an aerosol foam.

## SYNTHETIC "RED BLOOD CELLS"

This invention also provides for the manufacture of synthetic "red blood cells". The finished product is useful as a resuscitation fluid. In addition, the disclosed oxygen carrying composition is useful as a tissue and organ perfusion fluid; as a fluid useful in angioplasty and related procedures and as an oxygen carrying component in cell and tissue culture media.

Stroma-free hemoglobin is an essential component of the synthetic "red blood cells" of this invention. The "red blood cells" method includes hemoglobin derived from human sources and includes the use of hemoglobin derived from any pharmaceutically-acceptable source, including bovine and genetic engineering. It is preferred that the composition of this embodiment use stroma-free hemoglobin prepared by the method described below. If desired, modified hemoglobin, such as pyridoxilated-polymerized hemoglobin, lipsome-encapsulated hemoglobin and the like may be substituted for the preferred stroma-free hemoglobin. As another option, combinations of the hemoglobins described above may be used in this method. If genetic engineered, modified or other forms of hemoglobin preparations are used, it is preferred that enzymes, anti-oxidants and other components of red cell cytoplasm be added in amounts that will approximate normal, natural red cell blood concentrations in the finished "red cell" substitute. If preferred, the hemoglobin component used in this method may be lyophilized prior to use in the processing steps of this method.

The preferred size of the synthetic "red blood cell" of this invention is 1 micron or less. The finished "red cell" product is a coacervate-based matrix encapsulated by a coacervate-based film. The matrix contains stroma-free hemoglobin, antioxidants, enzymes and other components normally present in red cell cytoplasm. As an option, the formulator may add other compounds, such as a phospholipid; a sterol, such as cholesterol; and a protein, such as albumin or, preferably, coacervated egg albumin, during the formulation process. Other optional additives include anti-oxidants, other enzymes, electrolytes and other compounds normally present in human red cells. Such additions are made in quantities that approximate concentrations in the natural red cell or plasma.

The method used to prepare the synthetic "red blood cells" of this invention is as follows: (1) Hemolyze that amount of fresh or out-dated human red blood cells as will give about 800 mls of hemolyzed product and which, after the processing described below, will contain about 14 weight percent stroma-free hemoblogin. This hemoglobin concentration may be increased or decreased as preferred. Any conventional method to hemolyze red cells may be used. (2) Centrifuge the product of (1) at about 20,000 rpm for about 15 to 20 minutes. (3) Using a filter with pores of about 0.3 microns, filter the product of step (2) to obtain a solution of red blood cell cytoplasm. Discard the stroma and other debris trapped in the filter bed. (4) Add about 80 grams of polyethylene glycol (weight average molecular weight about 8000) 200 grams of dextrin, (weight average molecular weight about 19,500) to about 800 mls of the product of step (3) (the red cell cytoplasm solution). (5) Mix the product of step (4) using a Tissuemizer for about 20 to 30 minutes. (6) Filter the resulting product using a filter with pores of about 0.2 microns in diameter. Discard the residue in the filter bed and store the filtrate, preferably under refrigeration ($4°$-$10°$ C) for from 12 to 24 hours. (7) During the period of storage, the composition will separate into two phases - a coacervate phase and an equilibrium water phase. It is preferred that the period of storage be continued until no further separation of phases occurs. The phases are separated by means of a separatory funnel.

The upper, or coacervate phase will range from colorless to faint pink in color. The lower, or equilibrium water phase is generally red in color and contains all of the components present in the cytoplasm of natural human red blood cells. (8) At this stage, it is preferred that filtered egg yolk, in an amount ranging from aobut 0.1% to about 3% w/v, be added to the coacervate phase; about 1% w/v filtered egg yolk is preferred. As an option, any phospholipid may be substituted for the filtered egg yolk. Other options including the following: from about 0.5% to about 50% w/v sterol, preferably cholesterol, may be added to the coacervate phase and from about 0.5% w/v to about 10% w/v of filtered egg white may be added to the equilibrium water phase. In this option, any pharmaceutically-acceptable albumin may be substituted for the filtered egg white. (9) Next, process the product of step (8) in a Microfluidizer for about 20 minutes. The resulting product is a microemulsion composed of the equilibrium water phase containing the red cell cytoplasm components dispersed in the coacervate phase. The microemulsion comprises the synthetic "red cell" substitute which will function as a resuscitation fluid for humans and animals. As desired, the pH of the composition can be adjusted to about 7.4 using 1N HCl or NaOH depending upon the required adjustment. The viscosity of the product of step (9) is physiologically appropriate; however, it may be modified as an option to meet specific needs by the addition of normal saline solution or other appropriate physiologically-acceptable additives.

It is understood that in this application the term "resuscitation fluid" is used to mean a fluid that is designed to transport and offload oxygen and restore and maintain physiologically adequate oncotic pressures in the recipient of the product.

According to the practice of the present invention, the resuscitation fluid composition described above also will function as an organ and tissue perfusion fluid. As such, it can be used to provide oxygen and other physiologically important compounds, such as electrolytes and the like which are known to be useful to maintain viability of organs and tissues of human and other mammals stored prior to transplantation. The parameters of pH, viscosity, and electrolyte balance of the disclosed perfusion fluid may be adjusted as required by the formulator using the methods described above. The hemoglobin concentration of the perfusing fluid of this invention may be adjusted as required by increasing or decreasing the quantity of the red cell substitute in the perfusing fluid. As preferred, the oxygen carrying and releasing compounds and other useful compounds may be introduced into the disclosed perfusion fluid on a continuous or intermittent basis.

The resuscitation fluid described above also is useful to provide oxygen and, as preferred, other useful physiological compounds, i.e., drugs and the like, to patients undergoing cardiac surgery and angioplasty procedures.

The resuscitation fluid described above also is useful as a circulatory volume extender. In such application, the formulator may adjust the oxygen-carrying capacity of the composition by reducing the hemoglobin concentration of the composition to the preferred percent. In this embodiment, the principal aim is restoration of adequate blood pressure and volume.

Cell and tissue culture media can be prepared using the red blood cell substitute of this embodiment.

To prepare such media, disperse that quantity of the disclosed red cell substitute in about 100 mls of human or fetal serum, or other acceptable tissue culture media or combinations thereof, as will give the desired oxygen tensions, when oxygen is bubbled into the composition. In this application the red cell substitute is used in an amount that will comprise about 14 weight percent of hemoglobin. The hemoglobin concentration may be increased or decreased from 14 grams percent as required by the formulator.

## EXAMPLE 1

Disperse about 250 mgs. of erythromycin in a mixture comprised of filtered egg white and egg yolk contents of one egg. Mix that amount of melted glycerol mono-oleate as will comprise about 2% of the total weight of the preparation. Next, disperse the resulting composition in about 30 grams of filtered egg white and mix thoroughly. Next, process the composition for about 5 minutes in a colloid mill. During this processing step slowly add about 25 mls of ethyl alcohol to the product. This processing step will produce microencapsulated particles of erythromycin. The microencapsulated particles are washed with sterile water after which they are dried. The finished product may be used in a liquid or prepared as tablets, capsules, wafers and the like, for oral use.

## EXAMPLE 2

The method of Example 1 is followed except for the following: the product of Example 1 is dispersed in the filtered egg white of one egg. After this step, the preparation is processed in a colloid mill for about 10 minutes. During this processing step about 20 mls of ethyl alcohol is added dropwise. Following these steps the product is centrifuged, the particulate matter is separated and washed with sterile water and dried by any conventional technique comprising microencapsulated drug particles. The finished product may be used as an oral preparation using the desired dose in a conventional oral dosage form such as tablets, suspensions and the like.

## EXAMPLE 3

The method of Example 1 is followed except that 2% w/v of cholesterol is added to the product during the colloid mill processing step.

## EXAMPLE 4

Disperse 7.5 grams of erythromycin base in 4 grams of melted glycerol monostearate using a colloid mill to facilitate dispersion. Next, the resulting composition is mixed into 10 ml of filtered yolk of egg and 20 ml of distilled water and processed with a Tissuemizer for 20 minutes. Add 7.5 grams of erythromycin base into the resulting product and mix with a Tisuemizer for 15 minutes while adding 50 mls of 190 proof grain alcohol dropwise. Refrigerate the product for about 18 hours after which evaporate the alcohol from the product, such as with a Rotovapor. The composition comprises an oral form of erythromycin which can be used in conventional oral dosage forms.

## EXAMPLE 5

The method of Example 1 is followed except that 2% pilocarpine is used in place of erythromycin. In addition, the product is filtered to remove any particles larger than 0.5 microns. The product is dispersed in an isotonic solution after which the pH of the preparation is adjusted to about 7.4 using 1N HCl or NaOH as required. The finished product comprises a useful ophthalmic preparation.

## EXAMPLE 6

The method of Example 1 is followed except that 2% w/v of lecithin is added to the filtered egg yolk.

## EXAMPLE 7

The method of Example 2 is followed except that 2% pilocarpine is used in place of erythromycin and the size of the finished microencapsulated particles of pilocarpine are 0.5 microns or less. The finished product is dispersed in an isotonic solution after which, if necessary, the pH is adjusted to about 7.3 using either 1N, HCl or NaOH, as required. The finished product comprises an ophthalmic product.

## EXAMPLE 8

The method of Example 2 is followed except that the product of Example 2 is mixed into the egg white of one egg. The product is processed for about 25 minutes in a colloid mill during which about 35 mls of 180 proof ethyl alcohol is slowly added. Next, remove the alcohol by evaporation, wash the particles with sterile water and air dry. The product is comprised of microencapsulated particles of erythromycin. Process to remove all particles that exceed 0.5 microns. Mix 250 mgs of the product into 500 mgs of an albumin-lecithin based coacervate gel until the microparticles of erythromycin are evenly dispersed throughout the gel. The finished product comprises a composition useful as a tissue absorptive preparation.

## EXAMPLE 9

The method of Example 2 is followed except that 1% w/v of cholesterol is added during the colloid mill processing step prior to the addition of the ethyl alcohol.

## EXAMPLE 10

The method of Example 2 is followed except that a 20% solution of acetylcysteine is used in place of erythromycin. The finished product which is comprised of microparticles 1 micron or less is dispersed in physiological saline solution. The pH of the product is then adjusted to 7.3 after which it comprises a medication capable of adminstration via inhalation.

## EXAMPLE 11

The method of Example 10 is followed except that a combination of 10% acetylcysteine and 100 mgs of neomycin is used in place of acetylcysteine.

## EXAMPLE 12

To prepare a parenteral drug delivery composition, the following process is used: dissolve about 3 grams of carnuaba wax in about 225 mls of chloroform. Stir in 250 mgs of erythromycin while warming the preparation in a water bath at about 65-70° C until the composition comprises a clear yellow liquid. Add the egg yolk of one egg to the preparation and process in a colloid mill until a uniform dispersion is achieved. Next, add the egg white of one egg to the preparation and continue the colloid mill processing for 15 minutes. During this processing step, add about 250 mls of ethyl alcohol dropwise and also about .03 gm of methyl paraben in about 1 ml of polyethylene glycol during the colloid mill processing step. The product is filtered to remove all particles larger than 1 micron in size and lyophilized.

## EXAMPLE 13

To make a sustained release form of the parenteral product, disperse the product of Example 12 in the egg white of one egg. Next, process in a colloid mill for 15 minutes during which processing about 250 mls of ethyl alcohol is added dropwise. About .03 gms of methyl paraben in about 1 ml of polyethylene glycol is added during the colloid mill processing step. Disperse the product in any parenteral fluid; next the pH of the composition is adjusted to about 7.3. The product may be administered as a parenteral composition.

## EXAMPLE 14

The method of Example 2 is followed except that a 20% solution of acetylcysteine is used in place of erythromycin. The finished product which is comprised of microparticles 1 micron or less is dispersed in physiological saline solution. The pH of the product is then adjusted to 7.3 after which it comprises a medication capable of being administered via inhalation.

## EXAMPLE 15

The method of Example 14 is used except that a combination of 10% acetylcysteine and 100 mgs of neomycin is used in place of acetylcysteine.

## EXAMPLE 16

The method to prepare a synthetic "red cell" substitute is as follows: Hemolyze 850 mls of frozen human red cells. Follow this step by filtering the product to remove all red cell stroma. The filter bed should have pores of about 0.3 microns or less. Next, add 80 grams of polyethylene glycol (weight average molecular weight about 8000) 200 grams of dextrin, (weight average molecular weight about 19,500) to 800 mls of the red cell cytoplasm solution. Mix the product using a Tissuemizer for 30 minutes. Filter the resulting product using a filter with pores of about 0.2 micron in diameter. Discard the residue in the filter bed and store the filtrate, preferably under refrigeration at 4° C for about 24 hours. During the period of storage, the composition will separate into two phases. Separate the phases by means of a separatory funnel. The upper phase is referred to as the coacervate phase. The lower phase is referred to as the equilibrium water phase. Filtered egg yolk, preferably in an amount of about 1% w/v is added to the coacervate phase. Next, process the product in a Microfluidizer for about 20 minutes. The resulting product comprises a microemulsion composed of the equilibrium water phase containing the red cell cytoplasm components dispersed in the coacervate phase. The microemulsion comprises the synthetic "red cell" substitute.

## EXAMPLE 17

The method of Example 16 is followed except that 5% w/v of cholesterol is added to the coacervate phase.

## EXAMPLE 18

The method of Example 16 is followed except that 5% w/v of filtered egg white is added to the equilibrium water phase.

## EXAMPLE 19

The method of Example 16 is followed except that 1% w/v of urea is added to the product prior to the microfluidizing step.

## EXAMPLE 20

Melt 15 grams of glycerol mono-oleate at 75° C. Mix 6 grams of lidocaine into the melted glycerol mono-oleate using a glass rod. Cool the product to 30° C and add 6 mls of filtered egg yolk and process the product for 25 minutes in a colloid mill. Next, add 6 mls of filtered egg white to the product and process in a colloid mill for 15 minutes. Next, adjust the pH of the product to 8.0 using NaOH as required. Process the resulting product in a colloid mill for about 15 minutes adding 20 mls of 180 proof grain alcohol dropwise during this processing step. After this step, lyophilize the resulting product. The product is diluted with that quantity of sterile water as will make a 2% parenteral solution of lidocaine.

## EXAMPLE 21

The metohd of Example 20 is followed except that the lyophilized product of Example 20 comprises the starting material.

## EXAMPLE 22

The method to prepare an oral hematinic comprises the following steps: Mix 5 grams of lyophilized stroma-free hemoglobin into 10 grams of filtered egg white and process in a colloid mill for 20 minutes. Add 6 grams of filtered egg yolk to the product and process in a colloid mill for 10 minutes. Continue the colloid mill processing step for an additional 15 minutes while adding 50 mls of 180 proof ethyl alcohol dropwise. Filter the product using a filter bed with 0.3 micron pores; remove the precipitate from the filter bed and wash thoroughly with sterile water until all traces of alcohol have been removed. The composition comprises an oral hematinic which may be placed in any appropriate oral dosage form, i.e, capsules, in the desired dose, or prepared as a liquid, or the like.

## EXAMPLE 23

To produce parenteral delivery compositions for drugs, bioactive proteins and other medically useful compositions, the following procedures are used. Dissolve 0.1 gram of methyl paraben in 20 ml of distilled water heated to 80°C; dissolve 0.1 gram propyl paraben in 100 mls of distilled water heated to 100°C. Allow both preparations to come to room temperature. Next, (3) add 250 mgs of erythromycin in small increments to 60 ml of egg white; homogenize the product using a Tissuemizer for 30 minutes. Next, add 2.0 mls of the product of step (1) and 1.0 ml of the product of step (2) into the egg white and erythromycin composition. Next, homogenize the resulting product using a Tissuemizer for 10 minutes; during this step add 100 mls of 190 proof ethyl alcohol dropwise. Discard any foam that may develop on the preparation. The composition comprises microencapsulated particles containing erythromycin which can be placed in any injectable dosage form.

## EXAMPLE 24

The method of Example 23 is followed except that 5 mls of filtered egg yolk is added to the product prior to the finishing homogenizing step.

## EXAMPLE 25

Another useful variation of the method for preparing parenteral drug compositions comprises the following steps: (1) Mix 250 mgs of erythromycin in 45 ml of the coacervate phase from two phase coacervate composition prepared using 80 grams of propylene glycol E 8000; 200 grams of dextrin weight average molecular weight about 19,500 and 800 mls of distilled water. Process the composition in a Tisuemizer for 10 minutes; disperse 0.1 gm of methyl paraben and 0.01 gram of propyl paraben in 60 ml of filtered egg white and process the resulting composition in a Tissuemizer until a uniform composition is obtained. Next, add the parabens-egg white composition to the erythromycin-egg white composition preferably in small increments while homogenizing the composition with a Tissuemizer. During this processing step, add 100 mls of 190 proof grain alcohol dropwise to the product. Continue this step until a uniform suspension is produced. On completion of this step add 12 mls of filtered egg yolk and homogenize the resulting product using a Tissuemizer or other similar equipment. During this step adjust the pH to 7.4

## EXAMPLE 26

The following method is used to prepare an oral formulation: dissolve and mix 2 grams of ethyl cellulose in 50 ml of grain alcohol, 190 proof. Next, (2) dissolve 250 mgs of erythromycin in the minimum quantity of 190 proof grain alcohol necessary to dissolve the drug. This is done by dissolving the drug in the alcohol in increments of 5 mls or less until the drug is visually in solution. Mix the dissolved ethyl cellulose and the dissolved erythromycin using a Tissuemizer for 10 minutes. Next add 15 mls of egg white to the resulting product and homogenize using a Tissuemizer. Process the product of the previous step with lyophilizing equipment. The finished product comprises a useful oral dosage form of erythromycin.

## EXAMPLE 27

Dissolve 3 grams of carnuaba wax in 225 mls of chloroform. Stir in 250 mgs of erythromycin while warming the preparation in a water bath at 65-70°C until the composition comprises a clear yellow liquid. Add the egg yolk of one egg to the preparation and process in a colloid mill until a uniform dispersion is achieved. Next, add the egg white of one egg to the preparation and continue the colloid mill processing for about 10 to about 15 minutes. During this processing step, add about 250 mls of grain alcohol, 190 proof, dropwise and .03 gm of methyl paraben in 1 ml of polyethylene glycol. The product comprises microencapsulated coacervate-based matrix particles containing the drug. The product should be washed in distilled water until all traces of alcohol are removed. The product then is dispersed in 500 mls of saline solution and the pH of the composition is adjusted to about 7.3. The product then is lyophilized. It may be stored or formulated as a suspension or emulsion.

## EXAMPLE 28

To make a sustained release form of the product of Example 27, 250 mgs of the finished microencapsulated product is dispersed in the egg white of one egg, processed in a colloid mill for from 5 to 15 minutes during which processing step about 250 mls of ethyl alcohol is added dropwise. Also, .03 gms of methyl paraben in 1 ml of polyethylene glycol is added during this colloid mill processing step. After dispersal in physiological saline solution, the pH of the composition is adjusted to 7.4, lyophilized and stored until needed.

## EXAMPLE 29

Mix 20 grams of a commercial nutrition supplement (for example, VIVONEX®) in the egg white of one egg. The preparation then is processed in a colloid mill until the nutritional supplement is completely dispersed. Continue colloid mill processing until 200 mls of alcohol have been added dropwise to the preparation, i.e., about 15 minutes. The colloid mill processing continues for 5 additional minutes. Next, centrifuge the product for about 10 minutes at about 1500 rpm. Dissolve about 15 gms of type B gelatine, in water, heated to 40°C. Cool the gelatin to about 30°C. Add the precipitate from the centrifuging step to the gelatin and process the resulting product in a colloid mill for about 10 minutes. Next add 1 ml of methyl paraben solution to the product and refrigerate for about 24 hours. After this step, process the product in a colloid mill for about 30 minutes and then spray dry to produce finished microencapsulated particles of the nutrition supplement.

## EXAMPLE 30

Mix 10 mgs of the product of Example 2 into 1 gram of an albumin-lecithin coacervate-based gel. Mixing continues until there is an even distribution of the drug particles throughout the vehicle. Next, adjust the pH of the product to match that of the skin of the forearm. After pH adjustment, the composition is useful as a means to deliver the incorporated drug by transdermal method. Acceptable vehicles include creams, ointments, gels, and the like.

## Claims

1. A composition useful to introduce and/or transport one or more medically useful materials in the body of mammals by oral, tissue-absorptive transdermal, topical, inhalation or parenteral administration comprising a medically useful material contained in particles comprising biological material selected from the group consisting of egg white, egg yolk, parts, derivatives and synthetic equivalents of egg white, parts, derivatives and synthetic equivalents of egg yolk, and mixtures thereof.
2. The composition of claim 1, wherein the particles are particles of a coacervate-based matrix.
3. The composition of claim 1 or 2, wherein the particles have a structured surface.
4. The composition of anyone of claims 1 to 3, further including an encapsulating film over the particles.
5. The composition of claim 4, wherein the encapsulating film includes albumin.
6. The composition of claim 4, wherein the encapsulating film is formed from egg white.
7. The composition of anyone of claims 1 to 6, wherein the biological material is obtained from a non-fertile chicken egg.

8. The composition of anyone of claims 1 to 7, wherein the medically useful material is a fat-soluble drug.

9. The composition of anyone of claims 4 to 8, wherein the encapsulating film is hardened to permit appropriate release of the drug in the body of a recipient.

10. The composition of anyone of claims 4 to 9, wherein the encapsulating film includes a medically useful amount of a drug therein.

11. The composition of claim 10, wherein the drug in the encapsulating film is different than the drug in the egg yolk.

12. The composition of anyone of claims 2 to 11 which may be used as a red cell substitute or a hematinic comprising a coacervate-based matrix containing an oxygen carrier and optionally antioxidants, enzymes and other components normally present in red cell cytoplasm.

13. The composition of claim 12, wherein the oxygen carrier is selected from the group consisting of stroma-free hemoglobin, cross-linked hemoglobin, modified hemoglobin, other hemoproteins than hemoglobin, hemoglobin and other hemoproteins linked to carriers, hemoglobin and other hemoproteins contained in carriers, active components of hemoglobin polymers, heme of hemoglobin and combinations thereof.

14. The composition of claims 1 to 13, wherein the medically active material is selected from an antibiotic and a blood glucose lowering agent.

15. A method of preparing a composition of anyone of claims 1 to 14 comprising:

(1) preparing a mixture comprising an effective amount of a medically useful material and a material containing or consisting of a biological material selected from the group consisting of egg white, egg yolk, parts, derivatives and synthetic equivalents of egg white, parts, derivatives and synthetic equivalents of egg yolk, and mixtures thereof, to form an aqueous composition;

(2) optionally processing the aqueous composition in a colloid mill;

(3) optionally mixing a compound having dispersing, solubilizing and/or surfactant properties into the aqueous composition;

(4) processing the composition in a colloid mill;

(5) optionally adding one or more structuring and/or coacervating agents during the processing of step (4);

(6) optionally washing, drying and/or lyophilizing the product of step (5);

(7) optionally filtering the product to provide a composition having the desired particle size or particle size distribution.

16. The method of claim 15 further providing an encapsulating film comprising:

(8) mixing the product of claim 15 into material comprising egg white, a proteinaceous compound or a mixture thereof;

(9) processing the product of (8) in a colloid mill;

(10) optionally adding one or more structuring agents during step (9),

(11) optionally washing, drying and/or lyophilizing the product of step (10);

wherein steps (8) to (11) may be repeated as often as required.

17. The method of claim 16, wherein the product of claim 15 is mixed into the structuring agent of step (10) before steps (8 ) and (9) are carried out.

18. The method of anyone of claims 15 to 17, wherein the aqueous composition of (1) is constituted of the coacervate phase, the equilibrium water phase or both phases of a coacervate system.

19. The method of anyone of claims 16 to 18, wherein the encapsulating material contains the same medically useful material as the aqueous composition of (1) or a medically material different therefrom.

FIG-1-

Legend:
O—O 2.4 UNITS OF NEOPHORE INSULIN #1 (n=13)

●—● 2.4 UNITS OF NEOPHORE INSULIN #2 (n=6)

△--△ 2.4 UNITS OF UNENCAPSULATED INSULIN (n=3)

## FIG.2.

GLUCOSE BLOOD CONCENTRATION (mg/dL)

TIME (MINUTES)

□ INJECTED UNENCAPSULATED INSULIN  (n=10)

▼ UNENCAPSULATED INSULIN  (n=10)

○ ENCAPSULATED INSULIN  (n=10)